(19) Europäisches Patentamt
European Patent Office
Office européen des brevets

(11) **EP 4 684 813 A1**

(12) **EUROPEAN PATENT APPLICATION**

(43) Date of publication:
**28.01.2026 Bulletin 2026/05**

(21) Application number: **25190194.8**

(22) Date of filing: **17.07.2025**

(51) International Patent Classification (IPC):
**A61L 27/36** (2006.01)   **A61L 27/52** (2006.01)
**C12N 5/00** (2006.01)

(52) Cooperative Patent Classification (CPC):
**C12N 5/0068; A61L 27/3633; A61L 27/3687;
A61L 27/52; C12N 2533/90**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **22.07.2024 UA 202403745**

(71) Applicant: **Preci LLC
09100 Bila Tserkva (UA)**

(72) Inventors:
• **MOSEIKO, Vladyslav
Bila Tserkva (UA)**
• **KOVALCHUK, Sergiy
Bila Tserkva (UA)**

• **MOSHKIVSKA, Maria
Bila Tserkva (UA)**
• **SIMONOV, Andriy
Bila Tserkva (UA)**
• **OMELYANCHUK, Vladyslav
Bila Tserkva (UA)**
• **VOLOCHNIUK, Dmytro
Bila Tserkva (UA)**
• **GANOPOLSKYI, Anton
Bila Tserkva (UA)**
• **EFIMENKO, Konstantin
Bila Tserkva (UA)**

(74) Representative: **Barker Brettell LLP
100 Hagley Road
Edgbaston
Birmingham B16 8QQ (GB)**

(54) **METHODS OF PRODUCING A TISSUE DERIVED EXTRACELLULAR MATRIX HYDROGEL**

(57) The present disclosure relates to a method for producing an extracellular matrix hydrogel, the method comprising (a) decellularizing a tissue sample (105) to form a decellularized tissue mesh (115); (b) depolymerizing the decellularized tissue mesh to form a depolymerized protein dispersion (120), the depolymerizing comprising mixing the decellularized tissue mesh with a depolymerizing solution comprising a chaotropic agent to form a depolymerizing mixture; mechanically homogenizing the depolymerizing mixture at a chilled temperature to form a homogenized mixture; separating the homogenized mixture into (a) a waste precipitate and (b) a supernatant comprising the depolymerized protein dispersion; (c) filtering the depolymerized protein dispersion to form a sol; and (d) gelling the sol to form the extracellular matrix hydrogel (135), the gelling comprising combining the sol with a gelling solution comprising a kosmotropic agent; and separating a fluid phase away from the extracellular matrix hydrogel as it forms.

FIG. 1

EP 4 684 813 A1

## Description

FIELD OF THE DISCLOSURE

The present disclosure relates to methods of producing an extracellular matrix hydrogel derived from human or animal tissues for use in applications in cell culture and organ bioprinting.

CROSS-REFERENCE TO RELATED APPLICATIONS

This application claims priority to Ukrainian Patent Application No. a202403745 filed on July 22, 2024, the entire contents of which are hereby incorporated in their entirety by reference.

BACKGROUND

An extracellular matrix (ECM) is a complex polymeric network, characterized by its ambiguous physical state. In its liquid phase, the ECM can facilitate the diffusion of compounds while exhibiting varying degrees of structural dynamicity, solid-like rigidity, and viscoelasticity. When being used as the scaffold for a developing organism, the ECM can transition between organized solid structures and disordered fluid states, a dynamic critical for supporting morphogenesis.

Collagen, a primary component of mammalian ECM, forms a polymeric network through its triple helical structure. This polymeric network has a transient stability region at temperatures ranging at from 32 °C to 40 °C and at physiological pH, while melting above a certain temperature. The reconstitution of collagen in acid, followed by neutralization, leads to a sol-gel mixture having a critical gelation point at approximately 35 °C. Other constituents like fibronectin, elastin, and laminin, often incorporate polysaccharide fragments that contribute to the rigidity of the resulting network. Historically, collagen, basement membrane extracts, and various natural, semi-synthetic, and synthetic hydrogels have been used as ECM models. Hydrogels, which are polymers kinetically trapped in a colloidal state, advantageously encapsulate both the kinetic and thermodynamic aspects of ECM organization.

Currently, researchers utilize three types of hydrogels to mimic natural tissue scaffolds. These include Engelbreth-Holm-Swarm (EHS) mouse basement membrane extracts, synthetic polymers modified with collagen peptide residues, and pepsin-digested human-derived hydrogels. A problem with each of these approaches is they either do not include the initial extracellular matrix proteins or destroy the proteins. Further, none of these options replicate a natural ECM, as EHS-derived hydrogels have unnatural protein compositions compared to human tissue, synthetic polymers lack the complexity of natural tissue, and pepsin-digested human derived hydrogels have an altered primary structure of ECM proteins. There is a need for a sol-gel system extracted from human tissue that doesn't have modified native proteins and can replicate the structure, physicochemical properties, and biocompatibility of the human extracellular matrices.

SUMMARY

The present disclosure relates to a method for producing an extracellular matrix hydrogel. A method may include decellularizing a tissue sample to form a decellularized tissue mesh. Decellularizing may include mixing a tissue sample with a wash solution to form a decellularizing mixture, wherein a tissue sample is derived from a tumor having a fibrosis content ranging from about 40% to about 90%. Decellularizing may include separating a decellularizing mixture into a cellular waste fluid and a decellularized tissue mesh. A method may include depolymerizing a decellularized tissue mesh to form a depolymerized protein dispersion. Depolymerizing may include mixing a decellularized tissue mesh with a depolymerizing solution including a chaotropic agent and a surfactant to form a depolymerizing mixture. Depolymerizing may include mixing a decellularized tissue mesh with a depolymerizing solution including a chaotropic agent. A depolymerizing may include mechanically homogenizing a depolymerizing mixture at a chilled temperature to form a homogenized mixture. Depolymerizing may include separating a homogenized mixture into (a) a waste precipitate and (b) a supernatant comprising a depolymerized protein dispersion. In some embodiments, a waste precipitate may include a surfactant. A method may include filtering a depolymerized protein dispersion to form a sol. A method may include gelling a sol to form an extracellular matrix hydrogel. Gelling may include combining a sol with a gelling solution comprising a kosmotropic agent. Gelling may include separating a fluid phase away from an extracellular matrix hydrogel as it forms.

A method may include sterilizing a sol during a filtering. Sterilizing may include circulating a halogenated solvent in a permeate loop while a sol is maintained in a retentate loop while collecting a sterilized sol in a permeate loop. Sterilizing may include mixing a sol with a halogenated solvent. A method may include applying a vacuum to a sterilized sol. A method may include adding a hygroscopic agent to a gelling solution to modulate a stiffness of an extracellular matrix hydrogel. A tumor may include a sarcoma, a secondary liver cancer, a liver fibrosis, an osteosarcoma, a chondrosarcoma, a mixture of a heart fibrosis, a lung tissue and lung fibrosis, mesenchymal tissues and healthy organs, and combinations thereof. Separating a homogenized mixture into a waste precipitate and a supernatant may include centrifugation. Filtering of a depolymerized protein dispersion may be performed at a temperature of about 4°C. A method may include chloroform as a halogenated solvent. A halogenated solvent may be mixed with a sol at a concentration ranging from about 0.4 % v/v to about 3 % v/v. A halogenated solvent may be mixed with a sol for a time period ranging from about 3 hours to about 12 hours.

In some embodiments, mixing a tissue sample with a wash solution may include mixing the tissue sample and a wash solution at a rotation speed ranging from about 25,000 revolutions per minute to about 75,000 revolutions per minute, for a time of at least about 30 minutes. A wash solution may include about 0.34 M sodium chloride, about 1 mM ethylmaleimide, about 25 mM ethylenediaminetetraacetic acid disodium salt, and combinations thereof. Filtering a depolymerized protein dispersion may be done at a flow rate ranging from about 10 mL/min to about 70 mL/min and at a temperature ranging from about 0°C to about 8 °C. In some embodiments, a decellularized tissue mesh may have glycosylated protein tails, where a depolymerized protein dispersion derived from the decellularized tissue mesh may have at least about 95% of an amount of glycosylated protein tails in comparison to the decellularized tissue mesh, on a mass basis. A method may include preserving at least about 95% of glycosylated protein tails in a depolymerized protein dispersion with respect to a decellularized tissue mesh. A surfactant may include at least one of sodium dodecyl sulfate, cetyltrimethylammonium bromide, tween-20, tween-80 (polysorbate-80), triton X-100, and Pluronic F-127, and combinations thereof. A chaotropic agent may include at least one of sodium dodecyl sulfate, guanidinium chloride, guanidinium thiocyanide, urea, guanidine, thiourea, salts of calcium, nitrates, dithiothreitol, mercaptoethanol, tributyl phosphine, sodium borohydride, lithium perchlorate, lithium acetate, magnesium chloride, and combinations thereof.

A method may include applying a vacuum at a temperature ranging from about 0°C to about 8 °C for a time ranging from about 1 hour to about 3 hours. A hygroscopic agent may include a potassium citrate. An extracellular matrix hydrogel may have a stiffness ranging from about 1 kPa to about 250 kPa. A method may further comprise mechanically homogenizing a depolymerizing mixture at a temperature of about 4 °C. Filtering may include at least one of a double flow diafiltration, dialysis bag filtration, flow diafiltration, hollow fiber ultrafiltration, cartridge hollow fiber filtration, and cartridge tangential flow ultrafiltration. A yield of depolymerized protein dispersion may be at least about 70 mg/mL (depending on an original value can variate from 10 mg/mL for lipid-rich tissues to 110 mg/mL for heavily fibrotic tissue). A chilled temperature may range from about 0 °C to about 8 °C.

An ECM hydrogel formed by disclosed methods may have a primary human hepatocytes adhesion value ranging from about 1 kPa to about 10 kPa. An ECM hydrogel made by disclosed methods may have an FAK expression induction value ranging from about to 2-fold about 10-fold. An ECM hydrogel may be non-toxic to mice at working concentrations. An ECM hydrogel may include an orthotropic tumor growth rate ranging from about 10 mm$^3$/day to about 200 mm$^3$/day. An ECM hydrogel formed by disclosed methods extracellular matrix hydrogel may include a filtration buffer used during a filtering comprises at least one of water, a sodium dodecyl sulfate at concentration ranging from about 0.5% by volume to about 2% by volume, and a sodium chloride at a concentration ranging from about 0.1 M to about 4 M. Gelling may include raising a temperature of a sol from the temperature ranging from about 0°C to about 8 °C to a temperature ranging from about 26 °C to about 50 °C for a time period of at least about 1 minute.

In some embodiments, the present disclosures relates to a method for producing an extracellular matrix hydrogel. A method may include decellularizing a tissue sample to form a decellularized tissue mesh. Decellularizing may include mixing the tissue sample with a wash solution to form a decellularizing mixture, wherein the tissue sample is derived from a tumor having a fibrosis content ranging from about 40% to about 90%. Decellularizing may include separating the decellularizing mixture into a cellular waste fluid and a decellularized tissue mesh. A method may include depolymerizing the decellularized tissue mesh to form a depolymerized protein dispersion. A method may include filtering the depolymerized protein dispersion with a cartridge hollow fiber using a salt gradient to form a sol. A method may include gelling the sol to form the extracellular matrix hydrogel. Gelling may include (i) combining the sol with a gelling solution comprising a kosmotropic agent; and (ii) separating a fluid phase away from the extracellular matrix hydrogel as it forms.

In some embodiments, a method may include filtering the depolymerized protein dispersion with a cartridge hollow fiber using a salt gradient to form a sol. A salt gradient used in filtering may include a salt selected from sodium chloride, potassium chloride, zinc chloride, magnesium chloride, guanidinium chloride, and combinations thereof. The salt gradient of the filtering may include a concentration that ranges from about 0.001 M to about 1 M. The cartridge hollow fiber may include a porous filament ranging from about 5 kDa to about 200 kDa. In some embodiments, the filtering may be performed at a flow rate ranging from about 1 mL/min to about 1 L/min.

BRIEF DESCRIPTION OF THE DRAWINGS

The disclosure is illustrated by way of example, and not by way of limitation, in the figures of the accompanying drawings in which like reference numerals are used to refer to similar elements. It is emphasized that various features may not be drawn to scale, and the dimensions of various features may be arbitrarily increased or reduced for clarity of discussion.

FIG. 1 is a flow chart showing the production of an extracellular matrix hydrogel, according to some embodiments of the disclosure;

FIG. 2 is a flow chart showing a decellularizing of a tissue sample in a decellularizing reactor, according to some embodiments of the disclosure;

FIG. 3A is a photograph of a stained tissue sample that has not been decellularized, according to some embodiments of the disclosure;

FIG. 3B is a photograph of a stained decellularized tissue mesh, according to some embodiments of the disclosure;

FIG. 4 is a flow chart of a depolymerizing a decellularized tissue mesh to form a depolymerized protein dispersion, according to some embodiments of the disclosure;

FIG. 5 is a plot of guanidine HCl concentration over time, according to some embodiments of the disclosure;

FIG. 6 is a plot of the relationship of guanidine HCl after 600 minutes of dialysis, according to some embodiments of the disclosure;

FIG. 7 is a flow chart of a diafiltration reactor set-up for filtering a depolymerized protein dispersion to form a sol, according to some embodiments of the disclosure;

FIG. 8 is a flow chart of a tangential filtering process, according to some embodiments of the disclosure;

FIG. 9A is an image of a landscape of colloids formed after a diafiltration process and immobilized on a surface, according to some embodiments of the disclosure;

FIG. 9B is a plot of a particle height (y-axis) and diameter (x-axis) of an average of small colloids formed, according to some embodiments of the disclosure;

FIG. 9C is an image of a height corrected landscape of a gelled hSTS mixture and immobilized on a mica surface, according to some embodiments of the disclosure;

FIG. 9D is a photograph of a D-band calculation of formed fibers, according to some embodiments of the disclosure;

FIG. 10A is a plot showing a distribution of particles by number, according to some embodiments of the disclosure;

FIG. 10B is a plot showing a distribution of particles by intensity, according to some embodiments of the disclosure;

FIG. 11 illustrates various cell images, according to some embodiments of the disclosure;

FIG. 12A illustrates an orthotopic tumor progression in Balb/c mice having 4T1 subcutaneous tumors, according to some embodiments of the disclosure;

FIG. 12B illustrates a body weight progression of Balb/c mice having 4T1 subcutaneous tumors, according to some embodiments of the disclosure;

FIG. 13A illustrates an orthotopic tumor progression in SCID mice having MDA-MB-231 subcutaneous tumors, according to some embodiments of the disclosure; and

FIG. 13B illustrates a body weight progression of SCID mice having MDA-MB-231 subcutaneous tumors, according to some embodiments of the disclosure.

## DETAILED DESCRIPTION

The present disclosure relates to methods for producing an extracellular matrix (ECM) hydrogel. Disclosed methods produce hydrogels that advantageously have intact primary and secondary protein structures with respect to an original tissue sample source. Having intact primary and secondary protein structures permit disclosed ECM hydrogels to advantageously enable more accurate tissue modeling in comparison to known hydrogels. Disclosed methods produce ECM hydrogels having enhanced physiological properties that permit natural angiogenesis by artificial organs. Disclosed methods produce ECM hydrogels that are advantageously recognized by a host immune system, which is not a feature of known hydrogels. Being recognized by a host immune system increases susceptibility of disclosed hydrogels to be cleaved by host matrix metalloproteases, permitting them to naturally be removed by a host body. Additionally, human-derived hydrogels may increase cell attachment in different way than to the animal-derived or synthetic polymers, therefore promote the cells to adapt more native morphology and physiology. Those hydrogels may serve as a matrix for the complex cell based assays, as organ-on-chip or scaffold-based spheroid cultures.

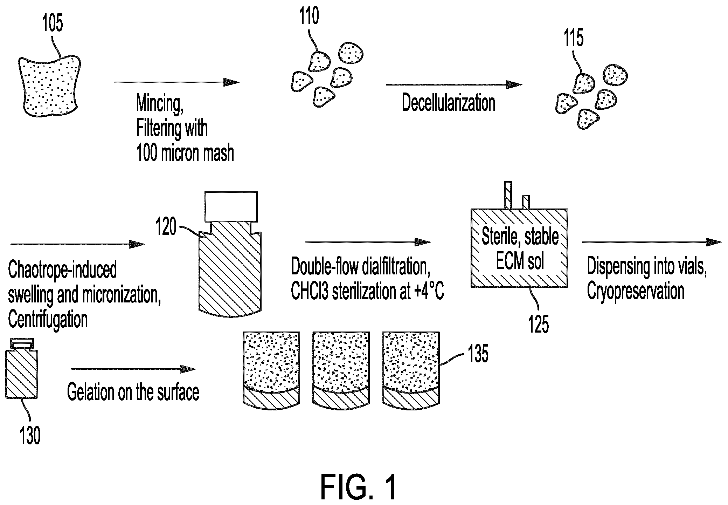

**FIG. 1** illustrates a disclosed method for producing an ECM hydrogel. As shown in FIG. 1, a disclosed method may include mincing and filtering a tissue sample 105 to produce a minced tissue **110**. A method may include decellularizing a minced tissue **110** to form a decellularized tissue mesh **115**. Methods disclosed herein may include depolymerizing a decellularized tissue mesh **115** by mixing the decellularized tissue mesh **115** with a depolymerizing solution containing a chaotropic agent, thereby forming a depolymerizing mixture. A method may include mechanically homogenizing a depolymerizing mixture to form a homogenized mixture, and then separating the homogenized mixture into a waste precipitate and a supernatant that contains a depolymerized protein dispersion **120**. As shown in FIG. 1, a method may include filtering a depolymerized protein dispersion **120**, such as by double-flow filtration, to form a sol **125**. Disclosed methods may include sterilizing a sol **125** with a halogenated solvent, such as chloroform. A sol **125** may be dispensed into vials **130** or any other known storage units. In some embodiments, a method may include gelling a sol **125** to form an ECM hydrogel **135**.

Harvesting and Storing Cancer-Derived Tissue

According to some embodiments, the present disclosure relates to methods of producing ECM hydrogels prepared from a tissue sample. Disclosed methods may include harvesting a tissue sample from a cancer-derived tissue. A cancer-derived tissue may be obtained from a human or an animal. In comparison to non-cancerous tissues, cancer-derived tissues may have a longer delivery window range after being harvested and before being processed, before they begin to degrade. Delivery window at +4°C is 2-10 hours at most.

Harvesting a tissue sample from a cancer-derived tissue may include excising, tearing, plucking, cutting, and any other know method of obtaining such a tissue. A tissue sample may have a delivery window ranging from about 4 hours to about 10 hours, under cold ischemic conditions. In some embodiments, a tissue sample may have a delivery window of about 4 hours, or of about 5 hours, or of about 6 hours, or of about 7 hours, or of about 8 hours, or of about 9 hours, or of about 10 hours, under cold ischemic conditions, where about includes plus or minus 0.5 hours. Cold ischemic conditions may include being at least partially submerged in a solution including phosphate buffer, Custodiol solution, Histidine-tryptophan-ketoglutarate (HTK) solution, Dulbecco's Modified Eagle Medium (DMEM), Roswell Park Memorial Institute (RPMI) with antibiotic solution, heparin sulfate, and others.

Methods disclosed herein may include examining a disclosed tissue sample for blood clots, lipid sediments, or other inclusions. In the event that blood clots, lipid sediments, and/or other inclusions are discovered, a disclosed method may include their surgical removal from a tissue sample. In some embodiments, disclosed methods may include harvesting a tissue sample having a high fibrosis content, so that the tissue sample is advantageously rich in ECM. For example, a method may include harvesting a tissue sample having a fibrosis content ranging from about 40 % to about 90%, or higher, by mass of a cancer-derived tissue. A fibrosis content of a tissue sample may be about 40 %, or about 50 %, or about 60 %, or about 70 %, or about 80 %, or about 90 %, where about includes plus or minus 5 %, by mass of the tissue sample. The measurement used in this particular work is based on the area estimate of the immunohistochemical slices.

In some embodiments, methods include harvesting a tissue sample from a tumor derived from a human or an animal. A tumor may include a sarcoma, a secondary liver cancer, a liver fibrosis, an osteosarcoma, a chondrosarcoma, mesenchymal tumors, carcinomas, a mixture of a heart fibrosis, a lung tissue and lung fibrosis, mesenchymal tissues and healthy organs, human soft tissue sarcoma (HSTS), and combinations thereof. A tumor, such as an HSTS, may advantageously include a high content of Collagens I, III, IV, fibronectin, elastin, glycosylated laminin, non-glycosylated laminin, and combinations thereof. A tumor may be obtained from fresh tissues (e.g., human or animal) or frozen tissues (e.g., human or animal). For example, prior to harvesting, a method may include freezing a tumor to a temperature of about 0°C, or about -10°C, or about -20°C, or about -30°C, or about -40°C, or about -50°C, or about -60°C, or about -70°C, or about -80°C, or below, where about includes plus or minus 5°C. A method may include freezing a tumor by placing into liquid nitrogen and then transferring the tumor to a freezer. In some embodiments, a tumor may be frozen without indication of degradation for at least about six months. A tumor may be minced before being frozen, which may advantageously enhance protein preservation.

Decellularizing a Tissue Sample

According to some embodiments, a method for producing an ECM hydrogel may include decellularizing a tissue sample to form a decellularized tissue mesh and a cellular waste fluid. Decellularizing may include mincing a tissue sample to form a minced tissue and then decellularizing the minced tissue to form a decellularized tissue mesh. A decellularized tissue mesh may include a mixture of water, proteins including collagen, and polysaccharides. A cellular waste may include removed cells. In some embodiments, residual cells not removed during a decellularizing may be detected within a decellularized tissue mesh. For example, residual cells may be detected by the residual nuclei or lipid inclusions, which have to be substantially removed to ensure the DNA-free and lipid-free colloid.

A method may include mincing a tissue sample to form a minced tissue having a size ranging from about 1 mm to about 10 mm, or bigger. For example, a minced tissue may have a size of about 0.1 mm, or about 1 mm, or about 2 mm, or about 3 mm, or about 4 mm, or about 5 mm, or about 6 mm, or about 7 mm, or about 8 mm, or about 9 mm, or about 10 mm, where about includes plus or minus 0.5 mm. Size of a minced tissue mesh may be measured in length, width, diameter, or any number of dimensions. A method may include letting a minced tissue stand at a temperature of about 25°C or colder, which may facilitate separation of a liquid fraction containing water, and solid fraction containing proteins and polysaccharides. In some embodiments, a solid fraction may be separated from a liquid fraction. A solid fraction may be weighed, with the weight being used to determine protein yields using standard calculations.

Disclosed methods may include mixing a tissue sample with a wash solution to form a decellularizing mixture. If a tissue sample is minced, a method may include mixing a minced tissue with a wash solution to form a decellularizing mixture. A decellularizing mixture may be separated into a cellular waste fluid and a decellularized tissue mesh containing proteins and polysaccharides. Mixing either a tissue sample or a minced tissue with a wash solution may be performed in a batch reactor, a flow reactor, or both. Combining either a minced tissue or a minced tissue with a wash solution may include maintaining a temperature ranging from about 0°C to about 35°C. For example, a temperature may be maintained at about 0°C, or about 5°C, or about 10°C, or about 15°C, or about 20°C, or about 25°C, or about 30°C, or about 35°C, where about includes plus or minus 2.5°C. A wash solution may include a salt including NaCl, KCl, LiCl, CsCl, CaCl$_2$, other salts, and mixtures thereof. A wash solution may include a salt at a concentration ranging from about 0.1 M to about 10 M, or higher. For example, a wash solution may include a salt at a concentration of about 0.1 M, or about 1 M, or about 2 M, or about 3 M, or about 4 M, or about 5 M, or about 6 M, or about 7 M, or about 8 M, or about 9 M, or about 10 M, where about includes plus or minus 0.5 M. Mixing a tissue sample or a minced tissue with a wash solution may be done at a rotation speed ranging from about 1,000 revolutions per minute (rpm) to about 80,000 rpm, or higher, and for a time of at least about 5 minutes. For example, mixing may be done at a rotational speed of about 1,000 rpm, or about 10,000 rpm, or about 20,000 rpm, or about 30,000 rpm, or about 40,000 rpm, or about 50,000 rpm, or about 60,000 rpm, or about 70,000 rpm, or about 80,000 rpm, where about includes plus or minus 5,000 rpm. A mixing may be performed for a time period of at least about 5 minutes, or at least about 10 minutes, or at least about 15 minutes, or at least about 20 minutes, or at least about 25 minutes, or at least about 30 minutes, or at least about 35 minutes, or at least about 40 minutes, or about 45 minutes, or about 50 minutes, or about 55 minutes, or about 6 minutes, where about includes plus or minus 2.5 minutes. In some embodiments, mixing may include a time ranging from about 1 hour to about 24 hours. For example, mixing may be performed for a time of about 1 hour, or about 4 hours, or about 8 hours, or about 12 hours, or about 16 hours, or about 20 hours, or about 24 hours, where about includes plus or minus 2 hours.

Decellularizing a tissue sample may include mixing a minced tissue with more than one wash solution. For example, decellularizing may include two wash solutions, three wash solutions, four wash solutions, five wash solutions, or more. Each of a wash solution may also include non-ionic surfactants. A non-ionic surfactant may include a Triton X, a Triton X-100, a Tween, a Tween 20, a Tween 80, a sodium dodecyl sulfate, a cetyltrimethylammonium bromide, a polysorbate-80, a Pluronic F-127, an ethylene diamine, ethylmaleimide, and combinations thereof. Other reagents may be included, such as ethylenediaminetetraacetic acid disodium salt (EDTA). Both non-ionic and ionic surfactants may be included at a concentration ranging from about 0.1 mM to about 1,000 mM, or higher.

Decellularizing a tissue sample may include centrifuging a decellularizing mixture to separate the decellularized mixture into a cellular waste fluid and a decellularized tissue mesh. Decellularizing a tissue sample may include filtering a decellularizing mixture to separate a decellularized tissue mesh from a cellular waste fluid. Decellularizing a tissue sample may include separating components of a decellularizing mixture in flow. In some embodiments, decellularizing a tissue sample may be iterative. For example, disclosed methods may include multiple cycles of combining a minced tissue with a wash solution to form a decellularizing mixture, then separating the decellularizing mixture into a cellular waste fluid and a decellularized tissue mesh. A decellularized tissue mesh may be combined with an additional wash solution and then subsequently separated. In some embodiments, ultrasonic, freeze-thaw, and other known decellularizing methods may be performed on a tissue sample.

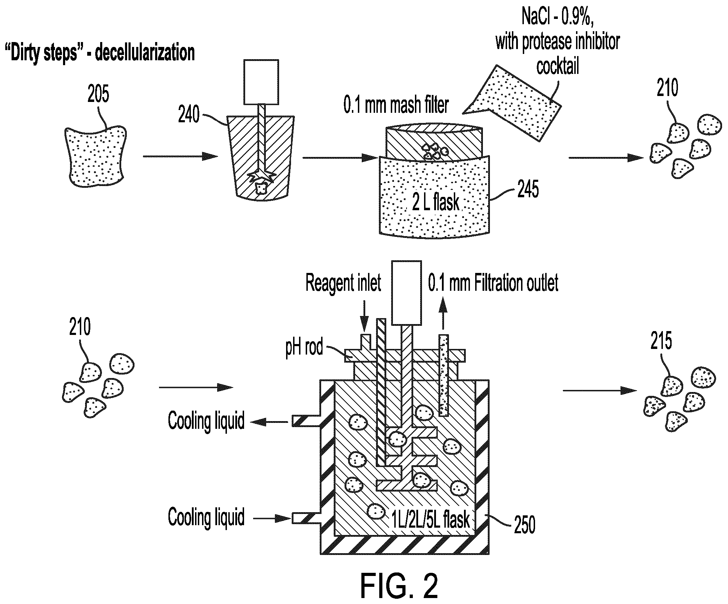

**FIG. 2** illustrates an embodiment of decellularizing a tissue sample **205** to form a decellularized tissue mesh **215**. As shown in FIG. 2, a tissue sample **205** may be placed into a blender **240** for a time period of about five minutes to form a blended tissue sample. A blended tissue sample may be placed in a filter **245** and rinsed with a 0.9% NaCl wash solution having a protease inhibitor. After being filtered and rinsed, a minced tissue **210** may remain and have a 2-3 mm in diameter may remain. A minced tissue **210** may be placed in a decellularization reactor **250** having a reagent inlet, a pH meter, a cooling liquid inlet, a cooling liquid outlet, and a filtration outlet. After being decellularized in a decellularization reactor **250**, a decellularized tissue mesh **215** may be isolated from a reactor.

In some embodiments, a decellularized tissue mesh **215** may have a size ranging from about 0.1 mm to about 10 mm, or larger. For example, a decellularized tissue mesh **215** may have a size of about 0.1 mm, or about 1 mm, or about 2 mm, or about 3 mm, or about 4 mm, or about 5 mm, or about 6 mm, or about 7 mm, or about 8 mm, or about 9 mm, or about 10 mm, where about includes plus or minus 0.5 mm. Size of a decellularized tissue mesh **215** may be measured in length, width, diameter, or any number of dimensions.

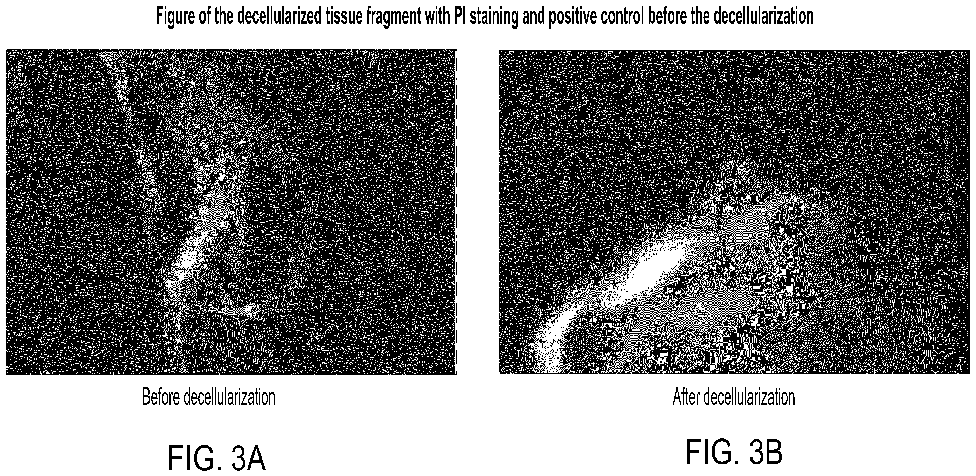

After decellularizing a tissue sample, a method may include staining a decellularized tissue mesh **215** with a stain to confirm decellularization, as shown in **FIGS. 3A and 3B.** FIG. 3A is a photograph of a tissue sample that is stained. FIG. 3B is a photograph of a decellularized tissue mesh that has been stained. A stain may include any known stain, including propidium iodide, which is used in FIG. 1 to show a difference between a tissue sample both before and after decellularization. A stain may also include crystal violet, methylene blue, 7-AAD, acridine orange, Calcein, carboxyfluorescein succinimidyl ester (CFSE), 4',6-diamidino-2-phenylindole (DAPI), Hoechst dyes, trypan blue, tetrazolium salts, and combinations thereof.

Further procedures will depend strongly on the mesh of the decellularized tissue. To assist the further decellularization the tissue may be additionally grinded using co-milling with dry ice. After the dry ice is left to evaporate the tissue mesh was shown to be 0.1-0.7 mm.

Depolymerizing a Decellularized Tissue

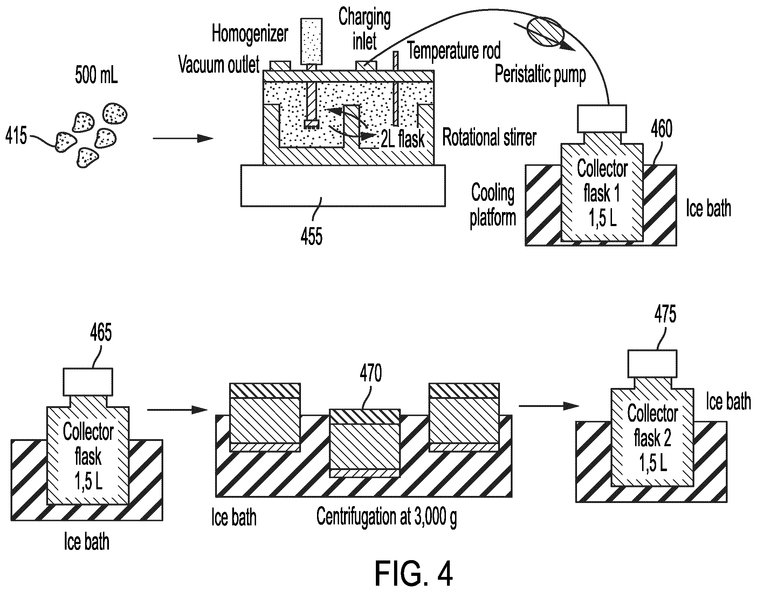

As shown in **FIG. 4**, According to some embodiments, a method for producing an ECM hydrogel may include depolymerizing a decellularized tissue mesh **415** to form a depolymerized protein dispersion **460**. Depolymerizing may include mixing a decellularized tissue mesh **415** with a depolymerizing solution to form a depolymerizing mixture. On this stage, principally only highly insoluble structural proteins (collagens, fibronectins and elastin) reside in the tissue, along with entrapped smaller proteins. A depolymerizing solution may include a chaotropic agent and a surfactant. As shown in FIG. 4, depolymerizing may occur in a depolymerization reactor **455**. A depolymerization reactor **455** may various components, including a vacuum outlet, a homogenizer, a charging inlet, a temperature rod, a rotational stirrer, and a cooling platform, as shown in FIG. 4. Within a depolymerization reactor **455**, disclosed depolymerizing may include mechanically homogenizing a depolymerizing mixture at a chilled temperature (e.g., less than 25°C) to form a homogenized mixture. Depolymerizing may include separating a homogenized mixture into (a) a waste precipitate containing a surfactant and (b) a supernatant containing a depolymerized protein dispersion, which may be collected in a collector flask containing the depolymerized protein dispersion **460**. Disclosed methods may advantageously form a depolymerized protein dispersion **460** having substantially preserved glycosylated protein tails with respect to a decellularized tissue mesh. Having substantially preserved glycosylated protein tails may provide for a final ECM hydrogel having an increasingly complex microenvironment. In some embodiments, depolymerizing may produce a depolymerized protein dispersion **460** having about 10 %, about 20 %, or about 30 %, or about 40 %, or about 50 %, or about 60 %, or about 70 %, or about 80 %, or about 90 %, or about 99 % of a glycosylated protein tails preserved with respect to a decellularized tissue mesh, where about includes plus or minus 5 %. In some embodiments maintaining glycosylated protein tails may ensure a proper viscosity (e.g. >10 millipascal second (mPa*s)) and elastic modulus (e.g., ranging from 1 kPa to about 100 kPa) for a formed disclosed ECM hydrogel. In some embodiments, having a proper viscosity and elastic modulus are essential for the cell-matrix recognition of disclosed ECM hydrogels. Conservation or removal of glycosylation protein tails (i.e., glycosylation sites) may be determined through orbitrap LC-MS of an initial tissue mesh and/or a depolymerized protein dispersion after the tissue mesh is dissolved by proteases and/or depolymerized. In some embodiments, gel-electrophoresis may indicate a conservation or removal of glycosylated protein tails of the depolymerized protein dispersion relative to a tissue mesh. In some embodiments, an acceptable glycosylated protein tail loss, by MW of the final protein of a depolymerized protein dispersion, may be within about 20% to about 80%, depending on the specifics of the procedure.

By mixing a decellularized tissue mesh **415** with a depolymerizing solution containing a chaotropic agent, polymeric proteins contained in a decellularized tissue mesh may be dispersed through chaotropic effects. A chaotropic agent may include sodium dodecyl sulfate, guanidinium chloride, guanidinium thiocyanide, urea, guanidine, thiourea, salts of calcium, nitrates, dithiothreitol, mercaptoethanol, tributyl phosphine, sodium borohydride, lithium perchlorate, lithium acetate, magnesium chloride, and combinations thereof. A chaotropic agent may be included in a depolymerizing solution at a concentration ranging from about 0.1 M to about 10 M, or higher. In some embodiments, solubility of a chaotropic agent may affect an upper boundary for a maximal concentration of the chaotropic agent included in a depolymerizing solution. For example, A chaotropic agent may be included in a depolymerizing solution at a concentration of about 0.1 M, or about 1 M, or about 2 M, or about 3 M, or about 4 M, or about 5 M, or about 6 M, or about 7 M, or about 8 M, or about 9 M, or about 10 M, where about includes plus or minus 0.5 M.

Depolymerizing a decellularized tissue mesh **415** to form a depolymerized protein dispersion **460** may be performed at a temperature ranging from about -10°C to about 25°C. For example, depolymerizing may be performed at a temperature of about -10°C, or about -5°C, or about 0°C, or about 5°C, or about 10°C, or about 15°C, or about 20°C, or about includes plus or minus 2.5°C. Disclosed methods may depolymerize a decellularized tissue mesh **415** while maintaining protein structural integrity, thus maintaining protein structural integrity (e.g., glycosylated protein tails) of proteins found therein. In some embodiments, disclosed methods may depolymerize a decellularized tissue mesh **415** while maintaining a temperature of below about 20°C, whereas known methods require temperatures of above 25°C, leading to loss of protein structural integrity.

A pH value of a depolymerizing solution may be maintained at a specific range during a disclosed depolymerizing of a decellularized tissue mesh **415**. A pH value of a depolymerizing solution may be maintained at a range of from about 5 to about 10. For example, a pH value of a depolymerizing solution may be about 5, or about 6, or about 7, or about 8, or about 9, or about 10, where about includes plus or minus 0.5. For example, a pH value of a depolymerizing solution may have a range of from about 6 to about 9.

A depolymerizing solution may include a surfactant during a depolymerizing. A surfactant may include a sodium dodecyl sulfate, a cetyltrimethylammonium bromide, a tween-20, a tween-80 (polysorbate-80), a triton X-100, a Pluronic F-127, and combinations thereof. A surfactant may be included in a depolymerizing solution at a concentration ranging from about 0.1% to about 10%, by percentage of the depolymerizing solution, or higher. For example, a surfactant may be included in a depolymerizing solution at a concentration of about 0.1 %, or about 1%, or about 2 %, or about 3 %, or about 4 %, or about 5 %, or about 6 %, or about 7%, or about 8 %, or about 9%, or about 10%, by percentage of the depolymerizing solution, where about includes plus or minus 0.5%. Surfactant maximal concentration is limited by the solubility of the surfactant in the depolymerizing mixture. A depolymerizing solution may also include buffers, including, but not limited to, a Tris-HCl, a phosphate buffered saline, a citrate buffer, and others. Disclosed depolymerizing solutions advantageously do not require protein digestion enzymes, such as pepsin, trypsin, and chymotrypsin. A depolymerizing solution may include, but do not require reducing agents, such as dithiothreitol, mercaptoethanol, $PBu_3$, and $NaBH_4$.

In some embodiments, depolymerizing may be performed in a depolymerizing reactor **455** having various components. For example, depolymerizing may be performed in a depolymerizing reactor **455** having one or more of an upperhead stirrer, a magnetic stirrer, mechanical disintegrator, a rotational frame stirrer, a mechanical homogenizer, a highpressure homogenizer, an ultrasonic homogenizer, a rotor-stator mechanism, a charging inlet for solid and liquid reagents, temperature jacked, ice batch, viscosimeter, pH measurement rod, and a temperature rod. A depolymerizing reactor **455** may be of various sizes ranging from 1 mL to about 10 L, or more. A depolymerizing reactor **455** may connected to a pump (e.g., peristaltic pump, vacuum pump) that may facilitate discharging of reactor contents once depolymerizing has been finished. Disclosed depolymerizing may be performed using multiple homogenization devices, such as by performing a simultaneous homogenization via both a mechanical disintegrator and a rotor-stator mechanism.

A disclosed method may include separating a homogenized mixture into a waste precipitate and a supernatant comprising a depolymerized protein dispersion **460**. Separating may include any known form, including batch filtering, centrifugation, decantation, and flow filtering. Separating may include separating any surfactants from a supernatant. Separating may provide for a depolymerized protein dispersion that is substantially clear of waste products **475**, as shown in FIG. 4. FIG. 4 illustrates how a collected depolymerized protein dispersion **460** may be separated via centrifugation **470** while cooling in an ice bath, then decanted into another collection flask containing a depolymerized protein dispersion that is substantially clear of waste products **475**. For example, a method may include separating a homogenized mixture into a supernatant and a waste precipitate containing protein aggregates. Separating may be performed at a temperature ranging from about -4°C to about 24°C. For example, separating may be performed at a temperature of about -4 °C, or about 0°C, or about 4°C, or about 8°C, or about 12°C, or about 16°C, or about 20°C, or about 24°C, where about includes plus or minus 2°C. Separating, in batch or in flow, may be performed at a flow rate ranging from about 1 mL/min to about 1 L/min, or more. Separating may be performed, in batch or in flow, at a rate of about 1 mL/min, or about 100 mL/min, or about 200 mL/min, or about 300 mL/min, or about 400 mL/min, or about 500 mL/min, or about 600 mL/min, or about 700 mL/min, or about 800 mL/min, or about 900 mL/min, or about 1,000 mL/min, where about includes plus or minus 50 mL/min. For example, separating may be performed in flow at a rate ranging from about 10 mL/min to about 70 mL/min at a temperature ranging from about 0°C to about 8°C.

According to some embodiments, a disclosed method may include analyzing a depolymerized protein dispersion formed by depolymerizing a decellularized tissue mesh. Analyzing may include measuring a yield, consistency of swelling, levels of protein hydrolysis, and other measurements. These metrics may be measured using known methods, including, but not limited to, size-exclusion chromatography, dynamic light scattering (DLS), atomic-force microscopy, nephelometry, ELISA, gel-electrophoresis, western-blot analysis, size-exclusion chromatography, 1H nuclear magnetic resonance spectroscopy (NMR), and 13C NMR. A yield of depolymerized protein dispersion may range from about 5 mg/mL to about 200 mg/mL. For example, a yield of depolymerized protein dispersion may be about 5 mg/mL, or about 20 mg/mL, or about 40 mg/mL, or about 60 mg/mL, or about 80 mg/mL, or about 100 mg/mL, or about 120 mg/mL, or about 140 mg/mL, or about 160 mg/mL, or about 180 mg/mL, or about 200 mg/mL, where about includes plus or minus 5 mg/mL. In some embodiments, yield may vary depending on a type of tumor tissue. For example, a yield may be at about 10 mg/mL for a lipid-rich tumor tissue or about 110 mg/mL for a heavily fibrotic tumor tissue.

Filtering a Depolymerized Protein Dispersion to Form a Sol

According to some embodiments, a method for producing an ECM hydrogel may include filtering a depolymerized protein dispersion to form a sol. Filtering may include filtering in batch, filtering in flow, double flow diafiltration, dialysis bag filtration, hollow fiber ultrafiltration, cartridge tangential flow ultrafiltration (TFF), cartridge hollow fiber filtration, and combinations thereof. During filtering, denatured proteins found within a depolymerized protein dispersion may repolymerize and refold, thereby permitting formation of a sol. Sol contains collagens, fibronectin, elastin and other structural proteins, which may depend on the exact tissue content.

Disclosed filtering may include exchanging solvents to form a stable sol. For example, a solvent having one salt may be exchanged for a solvent having a different concentration of the salt or even a different salt altogether. Exchanging solvents while filtering may prevent premature gelation, flocculation, and diffusion due to high viscosity. In some embodiments, a disclosed method may increase filtration speeds and incorporate in-line homogenization to prevent premature gelation, flocculation, and diffusion due to high viscosity. Besides changing one salt for another, solvent exchange may include changing a salt gradient, such as from 1 M NaCl to 0.15 M NaCl in water. Solvent exchanges may include exchanging one solvent type to another, such as from a NaCl in water solvent to a culture medium. For example, disclosed filtering may include a solvent exchange from a 6M guanidine HCl in water to a 3.4M NaCl in water, then to a 0.15 M NaCl, then to a culture medium. Another solvent exchange progression path may include a solvent exchange from a 6M guanidine HCl in water to a 0.15M NaCl in water, repurification of a 0.15 M NaCl in water, then exchange to a culture medium. In other instances solvent exchange may include other concentrations of NaCl, then specified here, surfactants, phosphate buffer saline, Tris-HCl buffer, sodium citrate buffer, sodium acetate buffer etc. Any known salt or culture medium may be used at any known concentration ranges. Besides solvent exchanging, disclosed filtering may include adding agitation to a filtration. For example, agitation may be added to a filtration being performed in a dialysis bag.

Filtering may include filtering a depolymerized protein dispersion through a membrane having various pore sizes. For example, a membrane may have a pore size ranging from 1 kDa to about 200 kDa, or greater. A membrane may have a pore size of about 1 kDa, or about 20 kDa, or about 40 kDa, or about 60 kDa, or about 80 kDa, or about 100 kDa, or about 120 kDa, or about 140 kDa, or about 160 kDa, or about 180 kDa, or about 200 kDa, where about includes plus or minus 10 kDa.

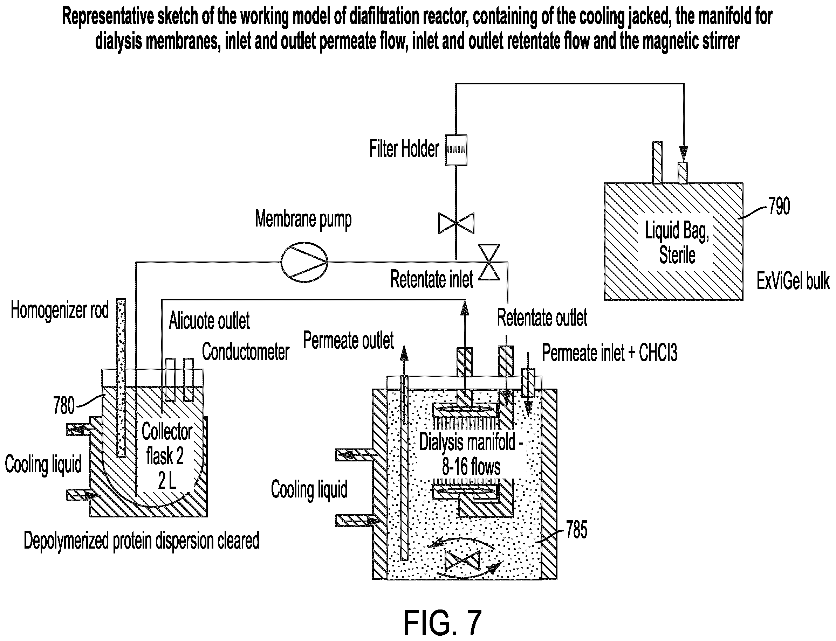

In some embodiments, filtering a depolymerized protein dispersion to form a sol may include centrifugal filtration, gravity filtration, bag filtration, reverse osmosis filtration, vacuum filtration, ultrafiltration, multilayer filtration, diafiltration, flow diafiltration, and combinations thereof. For example, as shown in **FIG. 7**, filtration may include, but is not limited to, flow diafiltration. Diafiltration may include multiple dialysis bag membranes placed in between manifolds. As shown in FIG. 7, flow diafiltration may include transferring a depolymerized protein dispersion from the collector flask into the sterile bag for the further preservation. In some embodiments filtration unit may be included between the collector flask and the collector bag.

Filtering a depolymerized protein dispersion to form a sol may include sterilizing the sol during the filtering. Sterilizing a sol during filtering may be performed in any way known in the art, including heating, treating with radiation, steam, treating with an electron beam, mixing with a solvent, and combinations thereof. Mixing with a solvent may include mixing with a halogenated solvent. For example, sterilizing may include circulating a halogenated solvent in a permeate loop so that it washes a sol, thereby removing unwanted contaminants. A halogenated solvent may include chloroform, methylene chloride, trichloroethylene, perchloroethylene, trichloro trifluoroethane, 1,1,1-trichloroethane, and mixtures thereof. A halogenated solvent may be circulated at a concentration ranging from about 0.1 % v/v to about 10 % v/v, by volume of either water, another solvent last exchanged, or by volume of the mixture of the sol and the halogenated solvent. For example, a halogenated solvent may be circulated at a concentration of about 0.1 % v/v, or about 1 % v/v, or about 2 % v/v, or about 3 % v/v, or about 4 % v/v, or about 5 % v/v, or about 6 % v/v, or about 7 % v/v, or about 8 % v/v, or about 9 % v/v, or about 10 % v/v, where about includes plus or minus 0.5 % v/v. Sterilizing a sol may include circulating a halogenated solvent in a permeate loop for a time period ranging from about 1 hour to about 24 hours. For example, a time period may be about 1 hour, or about 2 hours, or about 4 hours, or about 6 hours, or about 8 hours, or about 10 hours, or about 12 hours, or about 14 hours, or about 16 hours, or about 18 hours, or about 20 hours, or about 22 hours, or about 24 hours, where about includes plus or minus 1 hour.

After a sol is separated from a given solvent, a method may include applying a vacuum ranging from about 760 Torr to about $1 \times 10^{-12}$ Torr to the sol at a temperature ranging from about 4°C to about 24°C for a time ranging from about 0.1 hours to about 24 hours. A vacuum may be applied to a sol at a range of about 760 Torr to about 25 Torr (i.e., rough vacuum), about 25 Torr to about $1 \times 10^{-3}$ Torr (i.e., a medium vacuum), about $1 \times 10^{-3}$ Torr to about $1 \times 10^{-9}$ Torr (i.e., high vacuum), about $1 \times 10^{-9}$ Torr to about $1 \times 10^{-3}$ Torr (i.e., ultra-high vacuum). Applying a vacuum to a sol may be performed at a temperature of about -4°C, or about 0°C, or about 4°C, or about 8°C, or about 12°C, or about 16°C, or about 20°C, or about 24°C, where about includes plus or minus 2°C. Applying a vacuum may be performed at a time of about 0.1 hours, or about 1 hour, or about 2 hours, or about or about 4 hours, or about 6 hours, or about 8 hours, or about 10 hours, or about 12 hours, or about 14 hours, or about 16 hours, or about 18 hours, or about 20 hours, or about 22 hours, or about 24 hours, where about includes plus or minus 1 hour.

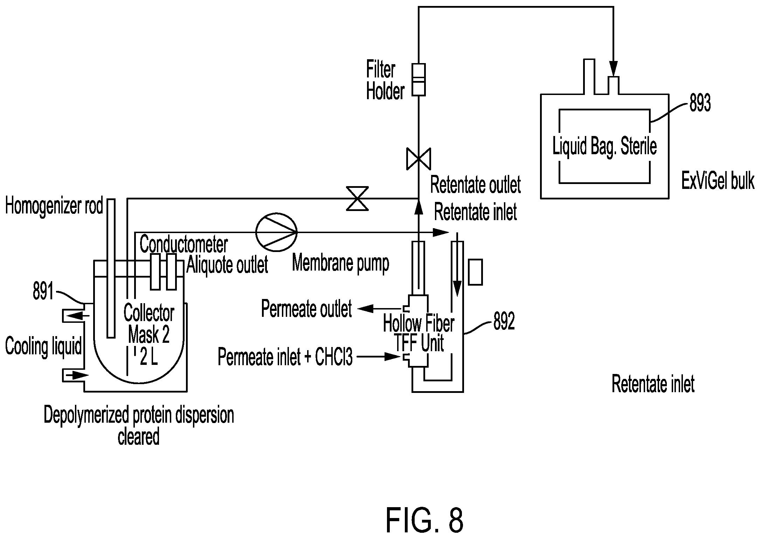

In some embodiments, as shown in **FIG. 8**, filtration may include, but is not limited to, a hollow-fiber tangential flow filtration. Diafiltration may include multiple dialysis bag membranes placed in between manifolds. As shown in FIG. 8, flow diafiltration may include transferring a depolymerized protein dispersion from a collector flask **891** into the sterile bag **893** for the further preservation. In some embodiments, a filtration unit **892** may be included between the collector flask and the collector bag. As shown in FIG. 8, a filtration unit may include a hollow-fiber filtration unit. A filtration unit **892** may include a permeate inlet so that a halogenated solvent (e.g., chloroform) may cycle through the filtration unit **892** and exit through a permeate outlet. In some embodiments, a filtration unit **892** may include a retentate outlet and a retentate inlet, so that samples for filtering may enter and exit.

A filtration unit **892** may have a pore size ranging from about 1 kDa to about 200 kDa. For example, a filtration unit **892** may have a pore size of about 1 kDa, or about 20 kDa, or about 40 kDa, or about 60 kDa, or about 80 kDa, or about 100 kDa, or about 120 kDa, or about 140 kDa, or about 160 kDa, or about 180 kDa, or about 200 kDa, where about includes plus or minus 10 kDa.

As shown in FIG. 8, a collector flask **891** may include a homogenizer rod, a conductometer, and an aliquot outlet for sampling contents. A collector flask **891** may include a cooling liquid inlet and outlet so that the contents of the collector flask **891** may be thermoregulated. For example, contents of a collector flask **891** may be cooled to a temperature of about 25°C, or about 20°C, or about 15°C, or about 10°C, or about 5°C, or about 0°C, or about -5°C, or about -10°C, or about -15°C, or about -20°C, or cooler, where about includes plus or minus 2.5°C.

In some embodiments, filtering may include filtering a depolymerized protein dispersion to form a sol may include filtering the depolymerized protein with a cartridge hollow fiber.

Filtering may include filtering the depolymerized protein with a cartridge hollow fiber using a salt gradient to form the sol. The salt gradient may include a salt including, but not limited to, lithium chloride, sodium chloride, potassium chloride, zinc chloride, magnesium chloride, guanidinium chloride, and combinations thereof. The salt gradient may include a salt concentration that ranges from about 0.001 M to about 1 M. For example, the salt gradient may include a salt concentration of about 0.001 M, or about 0.01 M, or about 0.1 M, or about 1 M, where about includes plus or minus 0.5 M. In some embodiments, the cartridge hollow fiber may include a porous filament ranging from about 5 kDa to about 200 kDa. For example, the cartridge hollow fiber may include a porous filament of about 5 kDa, or about 20 kDa, ,or about 40 kDa, or about 60 kDa, or about 80 kDa, or about 100 kDa, or about 120 kDa, or about 140 kDa, or about 160 kDa, or about 180 kDa, or about 200 kDa, where about includes plus or minus 10 kDa. Filtering may be performed at a flow rage ranging from about 1 mL/min to about 1 L/min. For example, the filtering may be performed at a flow rate of about 1 mL/min, or about 50 mL/min, or about 100 mL/min, or about 150 mL/min, or about 200 mL/min, or about 250 mL/min, or about 300 mL/min, or about 350 mL/min, or about 400 mL/min, or about 450 mL/min, or about 500 mL/min, or about 550 mL/min, or about 600 mL/min, or about 650 mL/min, or about 700 mL/min, or about 750 mL/min, or about 800 mL/min, or about 850 mL/min, or about 900 mL/min, or about 950 mL/min, or about 1,000 mL/min, where about includes plus or minus 25 mL/min.

In some embodiments, a hollow-fiber method may allow larger surface-to-volume ratio during the filtration, however, carries the significant disadvantage of the rapid fouling. Some fouling can be irreversible leading to the significant increase in production cost and need for the supply chain solutions for these devices. More rapid filtration is needed in cases when: a) depolymerized protein solution is too viscous, b) gelation of the protein occurs before the depolymerizing mixture is completely removed, c) flocculation of the protein occurs, when the GuaHCl concentration is lower than 1 M.

In some embodiments, all components used in a filtering step may be cooled to a temperature ranging from about 25°C to about -20°C, or cooler. For example, each component of a filtering step may be cooled to a temperature of about 25°C, or about 20°C, or about 15°C, or about 10°C, or about 5°C, or about 0°C, or about -5°C, or about -10°C, or about -15°C, or about -20°C, or cooler, where about includes plus or minus 2.5 °C.

Gelling a Sol to form an ECM Hydrogel

According to some embodiments, a method for using an ECM hydrogel may include gelling a sol to form an ECM hydrogel. Without removing the Gelling may include combining a sol with a gelling solution. A gelling solution may include a kosmotropic agent and a water. A kosmotropic agent may include an inorganic or organic salt of ammonium, a sodium salt, a sodium salt, a potassium salt, phosphate salt, polycarboxylate salt, a succinate salt, a citrate salt, an isocitrate salt. A kosmotropic agent may be included at a concentration ranging from about 0.01 M to about 0.15 M. For example, a kosmotropic agent may be included at a concentration of about 0.01M, or 0.03M, or 0.05M, or 0.07M, or 0.09M, or 0.11M, or 0.13M, or 0.15M.

Gelling may include raising a temperature of a sol from a temperature ranging from about 0°C to about 8°C to a temperature ranging from about 26°C to about 50°C for a time period of at least about 1 minute. For example, gelling may include raising a temperature of a sol from a temperature ranging from about 0°C to about 8°C to a temperature of about 25°C, or about 30°C, or about 35°C, or about 40°C, or about 45°C, or about 50°C, where about includes plus or minus 2.5°C. Gelling may include raising a temperature of a sol from a temperature ranging from about 0°C to about 8°C to a temperature ranging from about 26°C to about 50°C for a time period of at least 1 minute, or at least about 5 minutes, or at least about 10 minutes, or at least about 15 minutes, or at least about 20 minutes, or at least about 25 minutes, or at least about 30 minutes, or at least about 35 minutes, or at least about 40 minutes, or at least about 45 minutes, or at least about 50 minutes, or at least about 55 minutes, or at least about 60 minutes, or more, where about includes plus or minus 5 minutes. Gelling may include raising a temperature of a sol at a rate of about 1°C per minute, or about 2°C per minute, or about 3°C per minute, or about 4°C per minute, or about 5°C per minute, or about 6°C per minute, or about 7°C per minute, or about 8°C per minute, or about 9°C per minute, or about 10°C per minute, or about 11°C per minute, or about 12°C per minute, or about 13°C per minute, or about 14°C per minute, or about 15 °C per minute, or about 16°C per minute, or about 17°C per minute, or about 18°C per minute, or about 19°C per minute, or about 20°C per minute, or about 21°C per minute, or about 22°C per minute, or about 23°C per minute, or about 24°C per minute, or about 25°C per minute, or more, where about includes plus or minus 0.5°C per minute.

In some embodiments, an ECM hydrogel formed by disclosed methods may be preserved using shock preservation at a temperature ranging from -100°C to about 0°C. Shock preservation may include submerging an ECM hydrogel into a solvent/dry ice mixture. For example, the solvent may include an alcohol such as isopropanol. The solvent may include any known solvent generally mixed with dry ice, including acetone, ethanol, methanol, water, ethylene glycol, alkanes, other alcohols, and mixtures thereof. With the further transfer of the formed liquid to the -80°C freezer. For example, an ECM hydrogel may be preserved using shock preservation at a temperature of about -100°C, or about -80°C, or about -60°C, or about -40°C, or about -20°C, or about 0°C, where about includes plus or minus 10°C. Similarly, before forming a gel from a sol, gelation may be prevented while thawing a sol quickly by rapidly thawing the sol while stirring. Rapid thawing includes placing the vial of the frozen sol into the heated to +37°C water bath and further vigorous mixing of the sol. For example, a rapid thawing, or warming from a temperature of about -80°C to about 25°C, of a sol may be accomplished by stirring the sol at the temperature of the sol raises from about -80 °C to about 25°C.

A method for producing an ECM hydrogel may include gelling a sol to form an ECM hydrogel having a elastic modulus from about 1 kPa to about 250 kPa. In some embodiments, a thin ECM hydrogel may be formed by the steps of dilution of the ECM sol with the cell culture medium or buffer until the critical gelation concentration is reach and the gel does not form.

Biological Compatibility and Advantages of Disclosed ECM Hydrogels

A method for producing an ECM hydrogel may produce an ECM hydrogel having advantageous biological compatibility in comparison to known hydrogels. A method may form an ECM hydrogel having an elastic modulus ranging from 1 kPa to about 100 kPa. For example, a method may form an ECM with elastic moduli about 1 kPa, or about 2 kPa, or about 3 kPa, or about 4 kPa, or about 5 kPa, or about 6 kPa, or about 7 kPa, or about 8 kPa, or about 9 kPa, or about 10 kPa, where about includes plus or minus 0.5 kPa. In some embodiments, adhesion of primary cell cultures to a disclosed hydrogel may be higher in comparison to known hydrogels. For example, adhesion of primary cell cultures to a disclosed hydrogel may be about 10 % higher, or about 20 % higher, or about 30 % higher, or about 40 % higher, or about 50 % higher, or about 60 % higher, or about 70 % higher, or about 80 % higher, or about 90 % higher, or about 100 % higher, or more, in comparison to known hydrogels, where about includes plus or minus 5%. In some embodiments, confluency of a cell layer on a disclosed hydrogel may be higher in comparison to known hydrogels. For example, confluency of a cell layer on a disclosed hydrogel may be about 10 % higher, or about 20 % higher, or about 30 % higher, or about 40 % higher, or about 50 % higher, or about 60 % higher, or about 70 % higher, or about 80 % higher, or about 90 % higher, or about 100 % higher, or more, in comparison to known hydrogels, where about includes plus or minus 5%. For example, in experiments with human hepatocytes, isolated from the liver donor, confluency of a cell layer was higher by about 40% in comparison to know gels while having more similar to the human collagen I coating.

Disclosed methods may produce ECM hydrogels that induce immortal cell lines (e.g., HepG2) to advantageously have FAK expression induction values ranging from about to 1-fold about 20-fold, greater than known hydrogels. In some embodiments, FAK expression induction values may be measured using RNA sequencing. For example, a method may produce an ECM hydrogel having an FAK expression induction value of about 1 fold, or about 2 fold, or about 4 fold, or about 6 fold, or about 8 fold, or about 10 fold, or about 12 fold, or about 14 fold, or about 16 fold, or about 18 fold, or about 20 fold, higher than known hydrogels, where about includes plus or minus 1 fold.

Disclosed ECM hydrogels do not induce cytotoxicity in immortalized cell lines. For example, disclosed ECM hydrogels may not induce cytotoxicity in standard A549 and 4T1 cell lines. Toxicity may be assessed while staining cells with Propidium Iodide, Calcein-AM and DAPI and quantifying those images based on the imaging data.

A disclosed method may produce an ECM hydrogel that is non-toxic to preclinical animals. For example, no statistically significant body weight reduction, visual intoxication, visual idiosyncrasy may be observed in an animal (e.g., preclinical animal) while injecting a disclosed ECM hydrogel into the animal.

According to some embodiments, a disclosed ECM hydrogel may be used to develop a xenograph model using an injection (e.g., orthotropic injection) of a cell line (e.g., 4T1 cell line) into an animal (e.g., nude mouse) within or less than an equivalent time for a known hydrogel. A disclosed ECM hydrogel may be used to develop a xenograph model using an injection (e.g., orthotropic injection) of a cell line (e.g., A549 cell line) into an animal (e.g., nude mouse) within or less than an equivalent time for a known hydrogel. In some embodiments, a disclosed method may produce an ECM hydrogel having an orthotropic tumor growth rate ranging from about 1 mm$^3$/day to about 300 mm$^3$/day. For example, an ECM hydrogel may have an orthotropic tumor growth rate of about 1 mm$^3$/day, or about 20 mm$^3$/day, or about 40 mm$^3$/day, or about 60 mm$^3$/day, or about 80 mm$^3$/day, or about 100 mm$^3$/day, or about 120 mm$^3$/day, or about 140 mm$^3$/day, or about 160 mm$^3$/day, or about 180 mm$^3$/day, or about 200 mm$^3$/day, or about 220 mm$^3$/day, or about 240 mm$^3$/day, or about 260 mm$^3$/day, or about 280 mm$^3$/day, or about 300 mm$^3$/day, where about includes plus or minus 10 mm$^3$/day.

A vertical invasion assay may be used to monitor cell invasion of a disclosed ECM hydrogel, depending on a degree of compression of the formed ECM hydrogel gel. For this purpose the gel was diluted 1:2 with the cell culture medium in 96-well plate and centrifugated. For creation of elastic modulus gradient, the following regiments of 50-3000g might be used. The most compressed gel state forms when the mixture is centrifuged at the maximum rate. Following that the cells can be seeded on top of it and shot differential invasion into 0%-30% of the formed layer, depending on the density. In some embodiments, a similar analysis may not be performed with known hydrogels (e.g., Matrigel), because know hydrogels do not have a similar elasticity gradient. An elasticity gradient in human ECM may occur at during centrifugation due to the phase separation that occurs, , which may result from the water extrusion from the hydrogels. Compression of the hydrogel may range from about 50% to about 90%, by volume of the hydrogel, depending on the strength of the centrifugation applied. Known hydrogels, such as Matrigel, do not compress stably under similar centrifugation strengths applied to disclosed ECM hydrogels.

EXAMPLES

Example 1: Decellularization of a hSTS Tissue

A tissue sample was thawed at room temperature until it became fully softened. Weighing and selecting 250 grams of a tissue sample, with unused tissue sample being placed back into a freezer and frozen at -80 °C. In case a tissue sample was not processed immediately, it was stored in a refrigerator at +4 °C. A tissue sample was then cut into uniform pieces of 2-3 cm and placed in a blender. A tissue sample was ground with a large blender until it reached a size of 3-4 mm.

A ground tissue sample was placed in a beaker containing 4 liters of wash solution 1 (0.34 M NaCl, 1 mM ethylmaleimide, 25 mM EDTA-Na$_2$). A top-driven stirrer was immersed in a beaker, rotating at a speed of 50,000 revolutions per minute for 1 hour. A resulting sediment was filtered using a sieve with a pore size of 250 $\mu$m.

A ground tissue sample was then transferred to a beaker with 4 liters of wash solution 2 (1.3 M NaCl, 1 mM ethylmaleimide, 25 mM EDTA-Na$_2$). A top-driven stirrer was immersed in the beaker, rotating at a speed of 50,000 revolutions per minute for 1 hour. The sediment obtained was filtered using a sieve with a pore size of 250 $\mu$m.

The ground tissue sample was ground using an immersion blender to a size of 1 mm. The ground tissue was placed in a beaker with 4 liters of wash solution 3 (1.3 M NaCl, 1% w/v Triton X-100, 1% w/v chloroform, 1 mM ethylmaleimide). The top-driven stirrer was immersed in the beaker, rotating at a speed of 50,000 revolutions per minute for 1 hour. The sediment was filtered through a sieve with a pore size of 100 $\mu$m.

The ground tissue sample was then placed in a beaker with 2 liters of wash solution 4 (150 mM NaCl, 1% w/v triton X-100, 1 mM ethylmaleimide, 1% w/v chloroform). The top-driven stirrer was immersed in the beaker, rotating at a speed of 50,000 revolutions per minute overnight. The resulting sediment was filtered using a sieve with a pore size of 100 $\mu$m. The sediment was washed in wash solution 5 (150 mM NaCl) until foam formation ceased in the filtrate.

Example 2: Depolymerization of a Decellularized hSTS Tissue

A swelling solution containing: 6M Guanidine hydrochloride, 0.05 M Tris, 1% Tween, was cooled to a temperature of +4 °C. A chiller was cooled to -20 °C. A protein quantity was measured out ranging between 200-350 g was placed into a reactor. The swelling solution was measured and poured into the reactor at a ratio of 2 ml per 1 g of protein. Stirring was initiated in the reactor at a speed of 150 rpm. The temperature inside the reactor reached +4 °C. Denatured protein was added to the reactor and then mixed together with the remaining components. A mechanical homogenizer was turned on at 0.1 power. Gradually increasing the speed of the homogenizer, speed reached the value at which the temperature indicators of the protein solution remained stable over time and did not exceed +4 °C. The mixture was thoroughly homogenized using the disperser overnight at +4 °C to form a suspension.

A centrifuge was cooled to a temperature of +4 °C. The suspension was poured into centrifuge bottles and centrifuged at 3000 g for 30 minutes at +4 °C. The supernatant contained the target product. All steps were carried out on ice. The supernatant from all bottles were combined.

An efficiency of the swelling of the supernatant was evaluated based on the following tests: bicinchoninic acid assay with acceptable protein concentration between 8 to 110 mg/mL, volume of the precipitate (up to 20% of the total volume), Atomic Force Microscopy and Dynamic Light Scattering evaluation of the colloid size (<300 micron acceptable). No protein disaggregation >5% should be visible with Gel-Electrophoresis or Size-Exclusion Chromatography.

Example 3: Flow Dialysis

A flow dialysis system used herein may contain a manifold for dialysis membranes, a collector flask with cooling, a permeate inlet and outlet, retentate inlet and outlet, and a second collector flask with cooling. A concentration of GuaHCl was defined by permeate electrical conductivity measuring and transferring to concentration using calibration curve.

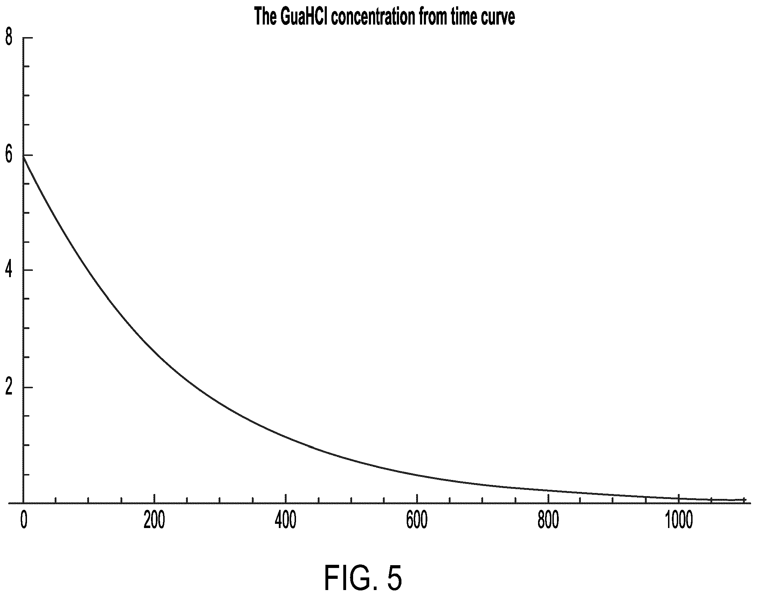

**FIG. 5** is a plot of the curve of GuaHCl concentration vs time.

Several hypotheses were made from this data, including that the GuaHCl diffusion rate does not depend from GuaHCl concentration, that the GuaHCl diffusion between retentate and permeate is in equilibrium process, that the dialysis speed depends on dialysis manifold surfaces area, and that the dialysis speed depends on collection flask volume and retentate exchange value. Based on these hypotheses kinetic mathematical modelling were made. Here are the equations that were used:

$$e(t) = \frac{C_0 S}{P + S + vt} \quad (1)$$

$$\frac{dU}{dt} \cdot S = -\frac{dZ}{dt} \cdot (P + vt) - Z(t)v \quad (2)$$

$$\frac{dZ}{dt} = \frac{Sk\left(U(t) - e(t)\right)}{P + vt} - \frac{Z(t)v}{P + Vt} \quad (3)$$

$$U(0) = 6, \quad Z(0) = 0$$

,

where $C_0$ = GuaHCl initial concentration; S = dialysis manifold common volume; t = time; v = permeate exchange flow speed; P = collection flask volume; k = reaction rate constant; U(t) = function of GuaHCl concentration in retentate decreasing; Z(t) = function of GuaHCl concentration in permeate increasing; and e(t) = equilibrium function.

To determine the reaction rate constant, the concentration of guanidine in the retentate was measured after a certain period. Then, for the equations above. the different values of k were fitted and values of U(t) at the time point, when the guanidine concentration at test sample was measured, determined. The value of U(t) that coincided with empirical determined value of guanidine concentration was chosen. In the cases where no values of U(t) coincided with empirical determined value of guanidine concentration, the values of k with more significant figures were fitted again.

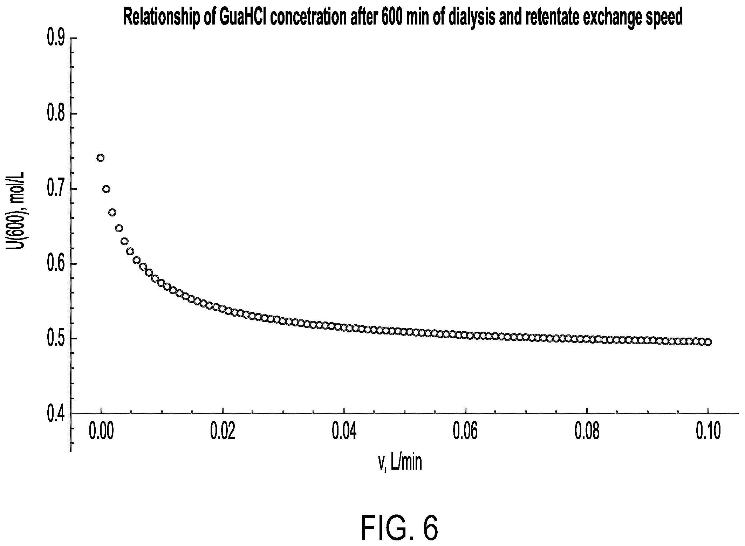

Flow dialysis conditions may be optimized based on knowing a rate constant as calculated above. Thus, permeate exchange flow speed can be determined as reaction rate constant by fitting. To visualize the relationship between GuaHCl concentration and permeate exchange flow speed the plot was built, as shown in **FIG. 6**. The plot from FIG. 6 shows that after 600 minutes interval of dialysis, the permeate exchange speed range from 0 L/min to 0.05 L/min is the most effective in terms of dialysis rate and permeate solution consumption.

High speed of mixing in the retentate loop may be required. Standard calculation of the mixing includes 1-3 volumes of the reactor exchange per minute. If the speed of mixing is decreased or the mixing is delayed during the process, irreversible gel formation is observed. Hollow fiber ultrafiltration was also efficient if similar principles are introduced. A pore size of 1 mm may be required for the process. Fast internal retentate flow may be required. Though, significant thread to this process is gelation inside the fiber, which significantly decreases the efficiency of the process. Hollow fiber diafiltration was efficient for the lower viscosity, low protein concentration hydrogels.

Example 4: Diafiltration of a Swelled Protein Mixture and Cryopreservation

As shown in FIG. 7, 21 cm of SnakeSkin™ 10 K MWCO dialysis tubing was cut, and connectors for dialysis manifold **785** were attached to each bag using two clamps on each side. Bags with an installed dialysis manifold **785** were connected to the dialysis setup. The internal loop of the dialysis setup was filled with air, and the integrity of all connections was checked.

The alignment of the dialysis bags was verified after inflation, ensuring that all bags were evenly inflated with air. Compression or twisting should be absent. Subsequently, the chiller was switched on at a temperature of -5 °C. 10 liters of cold dialysis solution (0.15M NaCl) were poured into the external loop of the dialysis setup, after which mixing in the external loop was initiated. The temperature in the external loop dropped to +4 °C. Then, the protein recovery vessel **780**, as shown in FIG. 7, of the dialysis setup was filled with depolymerized protein solution.

The internal pump was switched on at minimal power, and the protein solution was gradually added to the protein recovery vessel **780**, as shown in FIG. 7. The circulation speed of the protein was gradually increased to 5-10 volumes of the solution per minute. The volume of protein in the recovery vessel **780** should constitute 1/3. The conductivity sensor was placed in the protein recovery vessel **780**, along with a disperser set to minimum power. Ten liters of dialysis solution were prepared and placed in the refrigerator at +4 °C for further use.

The first dialysis cycle lasted for 6 hours, and the protein solution conductivity indicated less than 75 mS/cm of conductivity. After this, the dialysate in the external loop was replaced with cold dialysis solution, and 10 liters of dialysis solution were prepared for the next use. The second dialysis cycle lasted overnight, and the protein conductivity indicators were less than 20 mS/cm. The dialysate in the external contour was again replaced with cold dialysis solution, and another 10 liters of dialysis solution 2 (DMEM, 1% Chloroform) were prepared for the next stage. The third dialysis cycle lasted for 6 hours, and the protein conductivity indicators were 16 mS/cm. The dialysate in the external contour was again replaced with cold dialysis solution 2.

The fourth dialysis cycle lasted overnight. An aliquot of the protein solution (1 ml) was made for further quality control and frozen. Protein conductivity indicators should be less than 16 mS/cm. Optionally, afterward, a 0.45 $\mu$m inline filter was connected to the peristaltic pump. The pump with the filter was connected to the dialysis setup, and the protein solution was transferred to a sterile glass bottle **790**, as shown in FIG. 7. 1% antibiotic-antimycotic and 1% chloroform were added to the bottle.

The bottle **790** with the protein solution was moved to a shaker for 1 hour at +4 °C. Then, the bottle **790** was connected to a membrane vacuum pump at a pressure of 5 mBar for 1 hour at +4 °C, without removing it from the shaker. The bottle **790** was corked, placed on ice, treated with a disinfectant solution, and transferred to a clean area for further distribution. The work area was prepared for maximum cleanliness and sterility by treating all surfaces with a 70% ethanol solution. Subsequently, the automatic dispenser for filling underwent sterilization.

Bottles and their caps were also sterilized. For packaging volumes of 2 and 4 mL, cryotubes with a volume of 5 mL were used, while for packaging volumes of 10 and 20 mL, penicillin bottles of the corresponding volume were utilized. Labels were affixed to each bottle for batch identification. The bottles were placed in a stand and cooled in a refrigerator at a temperature of +4 °C.

A freezing solution was prepared in a polystyrene container, containing 2 volumes of dry ice and 1 volume of isopropyl alcohol. The bottle with the protein solution was kept on ice during use to prevent the formation of excess water in the external reservoir. Before packaging, the bottle with the protein solution was wiped dry from water droplets, treated with a disinfectant solution, uncorked, and connected to a dispenser. The dispensing volume was set on the dispenser.

Packaging was carried out in stages, with one stand of bottles per cycle. After taking a stand from the refrigerator, the protein mixture was quickly dispensed into the bottles. The bottles were corked and placed on a table. When using penicillin bottles, a crimper was employed. Each bottle was checked for tight sealing and correct labeling. Each bottle was immersed in the Freezing solution for shock freezing, and the procedure was repeated for the next stand. After waiting for the boiling of the Freezing solution with the bottles inside to conclude, the bottles with the packaged protein mixture were quickly removed using a large sieve with a handle and transferred to a freezer with a temperature of -80 °C. The packaged and shock-frozen protein mixture at -80 °C was considered the final product.

Example 5: Immobilization of the mica surface with ECM proteins

A solution (I) of APTES with a concentration of 0.0233 M in a solution of sodium hydroxide in methanol was prepared. That solution was mixed and stored at +4°C. Working solution was prepared. APTES solution (I) was diluted in methanol in the ratio n: 150, where n is the number of samples being prepared. Mica samples were placed in a tube with the working solution while stirring and at a temperature of 40 °C for 45 minutes. Mica samples were taken out, washed in methanol and dried. 20 microliters of glutaraldehyde were added to the mica samples. After 1 minute the samples were washed in methanol. The samples were cooled to 0°C. A drop of ECM protein dispersion was applied to a sample of mica, after 30 seconds wiped from the remaining solution with filter paper and washed with the large amount of water and wiped again.

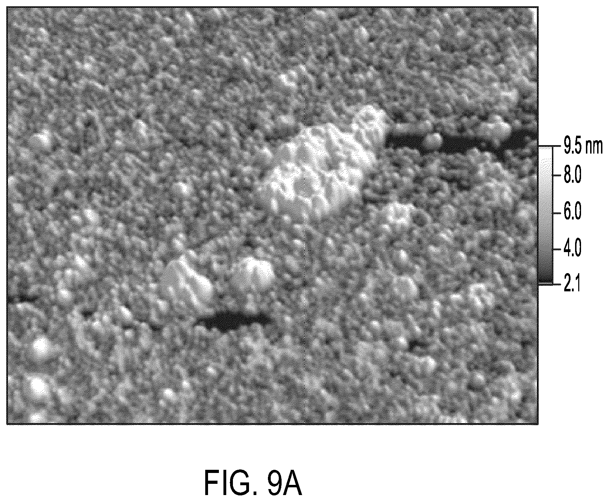

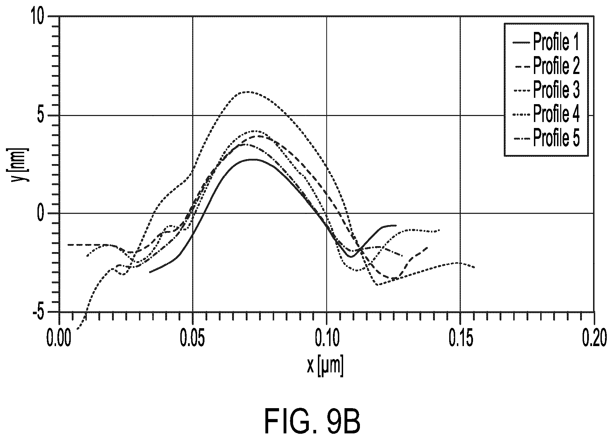

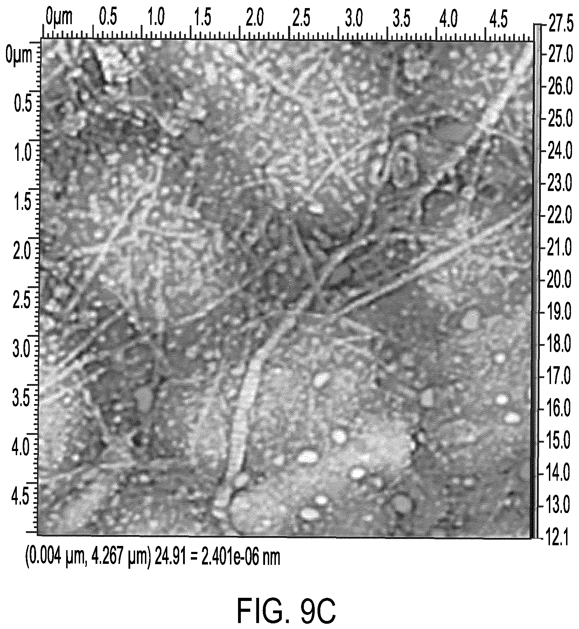

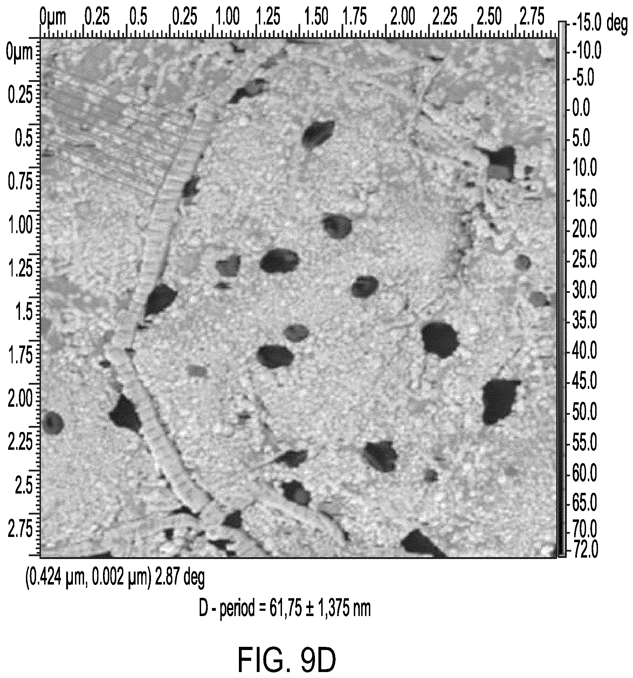

AFM studies (scanning probe microscope NanoScope IIIa Dimension 3000 tm (Bruker Corp.) for hSTS ECM sol showed that at +4°C the dominating particles are soft elastic colloids with the size of 50 nm - 100 nm. Sporadically particles with the size >200 nm occur (see **FIG. 9A**). FIG. 9B illustrates the particle height (y-axis) and diameter (x-axis) of the small colloids. After the gelation at +37°C long collagen fibers were observed, which constitute the aggregates with 0.5-2-micron size (See **FIG. 9C**). Those fibers themselves are formed by the characteristic bands of telocollagen (D-bands or D-periods). Those bands have a characteristic lengths of collagen D-band (61,75$\pm$1,375 nm), as shown in **FIG. 9D**.

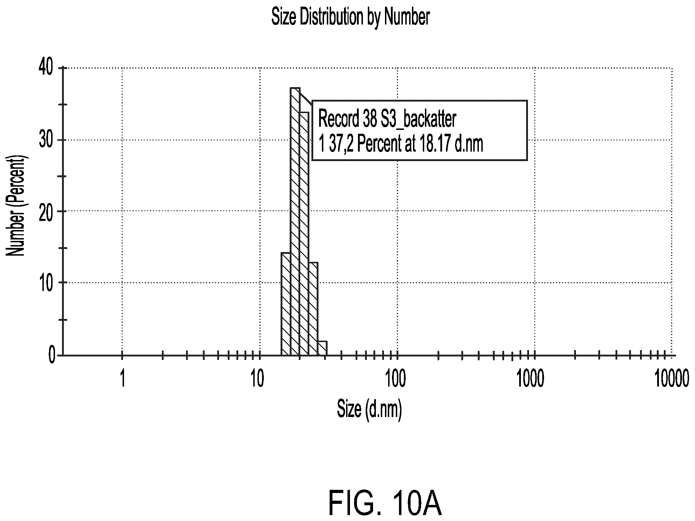

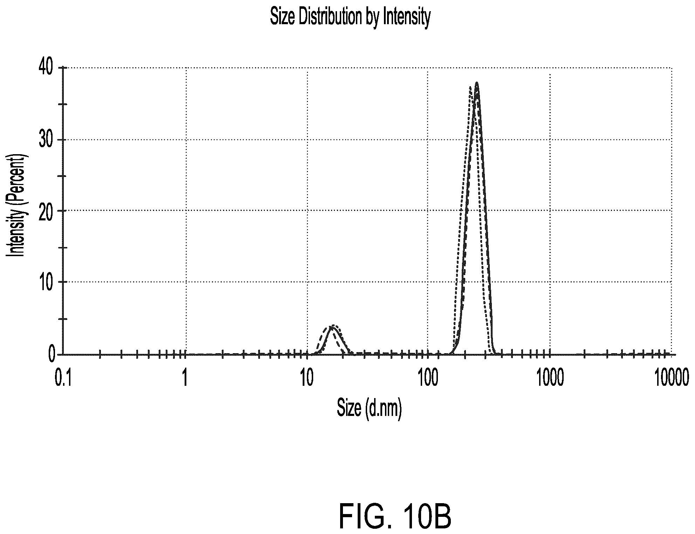

DLS studies for hSTS ECM sol (Zetasizer nano ZS) at +4°C showed domination of the 10-30 nm particles in the solution (see **FIG. 10A**). This is mostly correlated to the AFM data. The mean shift from 75 nm to 20 nm is possibly attributed to the coalescence of the sol particles on the mica surface. Larger particles 250 nm and more are also present in the solution at +4°C in the number lower than 250 nm, however, their scattering intensity (see **FIG. 10B**) is significantly higher. This is the possible explanation for the turbidity of the mixture.

Data for other tissue origin is comparable with those findings. As the general phenomenon, percolation of the hECM sol occurs due to the formation of the collagen fibers from the colloids of 10-50 nm in size. Critical viscosity (gelation) is achieved by the formation of the collagen fiber network and partial aggregation of those fibers into the 0.5-2 micrometer particles.

Example 6: Coating of a 96 Well-plate with a hECM Hydrogel

A hECM hydrogel shot of 5 mL was taken out of -80 °C freezer. The hydrogel shot was placed into the ice bath and mixed from time to time carefully. After the small amount of ice remained in the mixture, the mixture was stirred vigorously with the 1 mL micropipette until homogeneous. The formed solution may be stored at + 4°C for 24 hours. A solution was formed by diluting 1/20 - 1/10 for the thin-layer hydrogel coating and 1/4 - 1/2 for the thick-layer coating with the cold culture medium. The solution was dispensed at 30 microliters/well while keeping the solution and the well-plate cold. The mixture was then centrifugated at 2000 g for 10 minutes at +4°C and cured at +37°C inside the incubator. Formed upper fluid level was removed. The cells were seeded on top of the layer.

Seeded cells of HepG2 (ATCC) on the surface of the thin gel displayed the phenotypical differences between from the Corning Matrigel® coating and tissue-culture plastic. Despite that, cells seeded within the thin-layer hydrogel displayed similar colony formation rates. This shows the unique nature of this material as not only the solid support but also the biological environment that can result in the different cell properties than those with commercially available products.

To develop the allograph model, using hECM 4 T1 cells were orthotopically inoculated into 4th right mammary fat pad. Cells had been cultured in DMEM (4.5 g/L glucose) with 10 % FBS, 100 U/mL of penicillin, and 100 µg/mL of streptomycin at 37°C and 5 % CO2. Cells were harvested using 0.05 % trypsin-EDTA solution, centrifuged, and suspended in serum-free DMEM. Cells count and viability were accessed using a hemacytometer and trypan blue exclusion test. The final cells suspensions of $10 \times 106$/mL in DMEM and Matrigel from Corning or hECM mixed (3:1), were kept on ice before injection.

To develop the xenograph model, using hECM 5 M of MDA-MB-231 cells were orthotopically inoculated into 4th right mammary fat pad of the mouse. Cells had been cultured in RPMI (4.5 g/L glucose) with 10 % FBS, 100 U/mL of penicillin, and 100 µg/mL of streptomycin at 37°C and 5 % CO2. Cells were harvested using 0.05 % trypsin-EDTA solution, centrifuged, and suspended in serum-free DMEM. Cells count and viability were accessed using a hemacytometer and trypan blue exclusion test. The final cells suspensions of $50 \times 106$/mL in DMEM and Matrigel from Corning or hECM mixed (1:1), were kept on ice before injection.

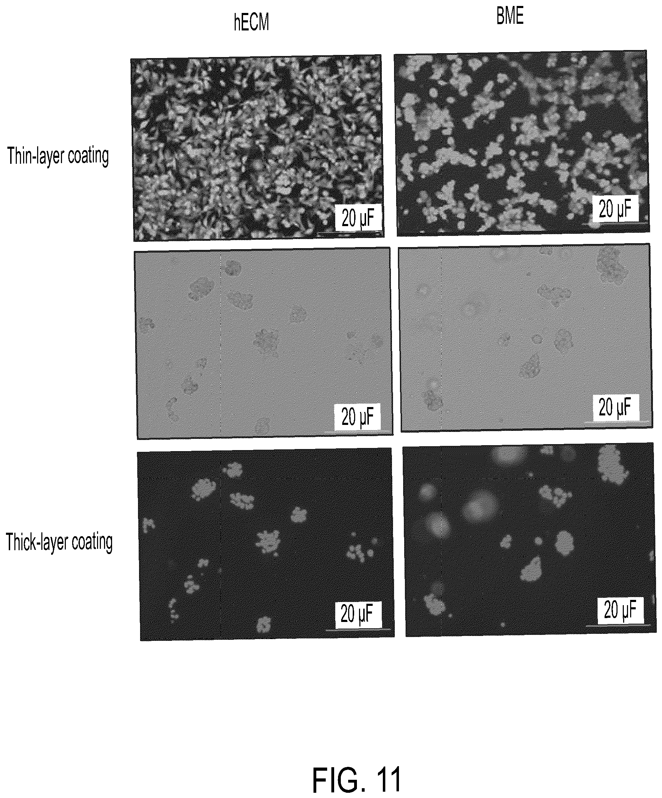

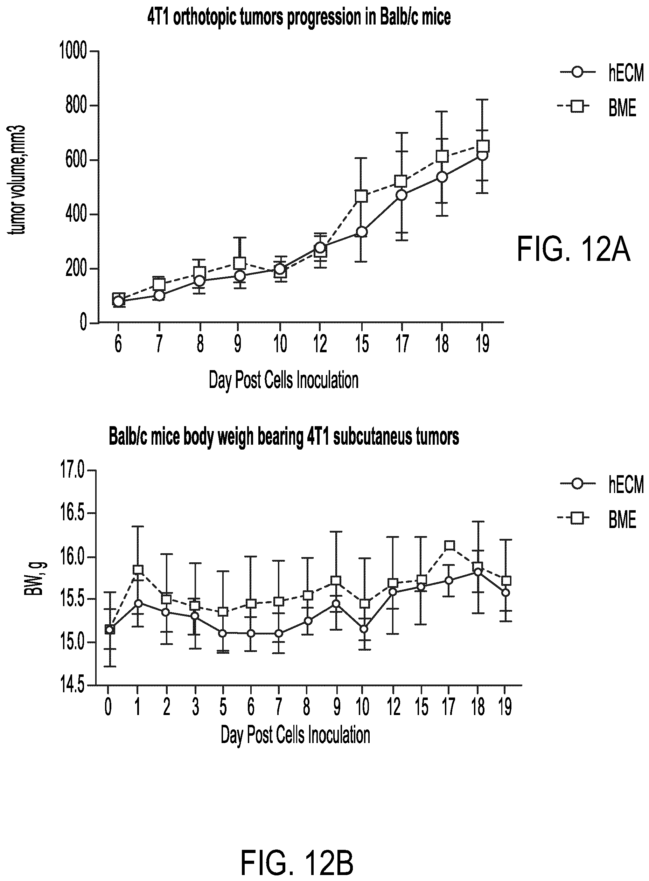

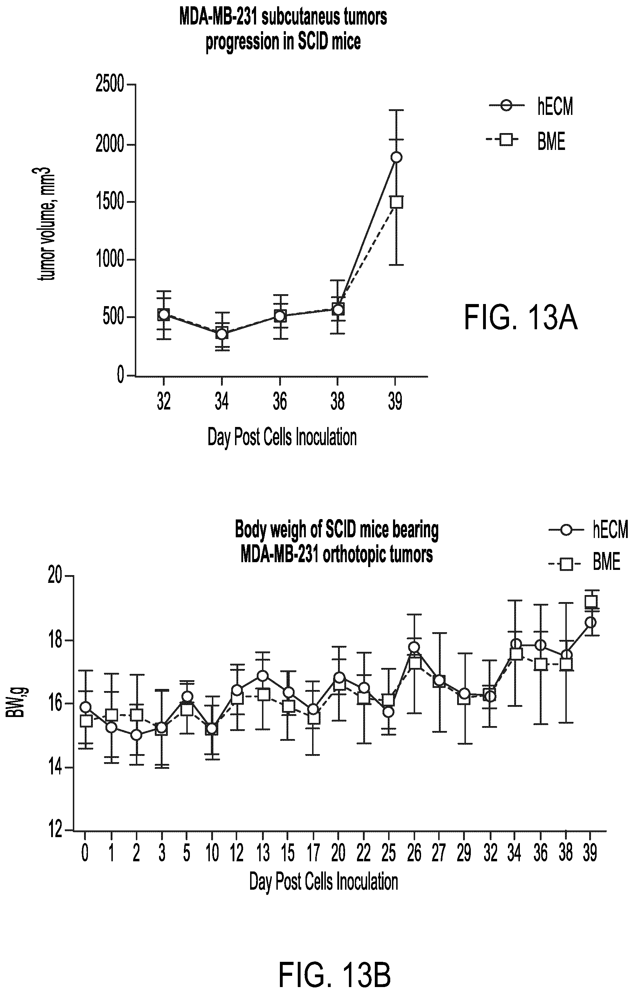

Overall, analogous to the thick-layer coating experiments mice did not show any signs of toxicity and the growth rate resembled that of the Corning Matrigel® products. Though, there were significant phenotypic alteration towards more matrix-adhesive cell structure. FIG. 10 includes photographs of images comparing the hECM hydrogels prepared using disclosed methods to the Corning Matrigel® products (BME). **FIG. 11** shows an increase in differentiation due to a higher attachment of the HepG2 cells on thin-layer coating (Calcein AM, Propidium Iodide staining) and colony formation assays in comparison to the thick-layer hydrogels. **FIGS. 12A** and **12B** illustrate orthotopic tumor progression in mice as well as the body weight of Balb/c mice baring 4T1 subcutaneous tumors, whereas the disclosed hECM hydrogels exhibit a similar performance as the BME hydrogels. Similarly, **FIGS. 13A** and **13B** illustrates orthotopic tumor progression in mice as well as the body weight of SCID mice baring MDA-MB-231 subcutaneous tumors, whereas the disclosed hECM hydrogels exhibit a similar performance as the BME hydrogels.

Reference in the specification to "one implementation" or "an implementation" means that a particular feature, structure, or characteristic described in connection with the implementation is included in at least one implementation of the disclosure. The appearances of the phrase "in one implementation," "in some implementations," "in one instance," "in some instances," "in one case," "in some cases," "in one embodiment," or "in some embodiments" in various places in the specification are not necessarily all referring to the same implementation or embodiment.

Finally, the above descriptions of the implementations of the present disclosure have been presented for the purposes of illustration and description. It is not intended to be exhaustive or to limit the present disclosure to the precise form disclosed. Many modifications and variations are possible in light of the above teaching. It is intended that the scope of the present disclosure be limited not by this detailed description, but rather by the claims of this application. As will be understood by those familiar with the art, the present disclosure may be embodied in other specific forms without departing from the spirit or essential characteristics thereof. Accordingly, the present disclosure is intended to be illustrative, but not limiting, of the scope of the present disclosure, which is set forth in the following claims.

**Claims**

1.   A method for producing an extracellular matrix hydrogel, the method comprising:

    (a) decellularizing a tissue sample to form a decellularized tissue mesh, the decellularizing comprising:

        (i) mixing the tissue sample with a wash solution to form a decellularizing mixture, wherein the tissue sample is derived from a tumor having a fibrosis content ranging from about 40% to about 90%; and
        (ii) separating the decellularizing mixture into a cellular waste fluid and a decellularized tissue mesh;

(b) depolymerizing the decellularized tissue mesh to form a depolymerized protein dispersion, the depolymerizing comprising:

(i) mixing the decellularized tissue mesh with a depolymerizing solution comprising a chaotropic agent to form a depolymerizing mixture;
(ii) mechanically homogenizing the depolymerizing mixture at a chilled temperature to form a homogenized mixture;
(iii) separating the homogenized mixture into (a) a waste precipitate and (b) a supernatant comprising the depolymerized protein dispersion;

(c) filtering the depolymerized protein dispersion to form a sol; and
(d) gelling the sol to form the extracellular matrix hydrogel, the gelling comprising:

(i) combining the sol with a gelling solution comprising a kosmotropic agent; and
(ii) separating a fluid phase away from the extracellular matrix hydrogel as it forms.

2. The method for producing the extracellular matrix hydrogel according to claim 1, further comprising:

sterilizing the sol during the filtering, wherein the sterilizing optionally comprises mixing the sol with a halogenated solvent, the method optionally further comprising applying a vacuum to the sterilized sol, optionally applying the vacuum at a temperature ranging from about 0°C to about 8°C for a time ranging from about 1 hour to about 3 hours.

3. The method for producing the extracellular matrix hydrogel according to claim 1 or claim 2, further comprising:
adding a hygroscopic agent to the gelling solution to modulate the stiffness of the hydrogel.

4. The method for producing the extracellular matrix hydrogel according to any one of claims 1 to 3, wherein the tumor comprises a sarcoma, a secondary liver cancer, a liver fibrosis, an osteosarcoma, a chondrosarcoma, a mixture of a heart fibrosis, a lung tissue and lung fibrosis, mesenchymal tissues and healthy organs, and combinations thereof.

5. The method for producing the extracellular matrix hydrogel according to any one of claims 1 to 4, wherein at least one of:

the mixing the tissue sample with the wash solution comprises mixing the tissue sample and the wash solution at a rotation speed ranging from about 25,000 revolutions per minute to about 75,000 revolutions per minute, for a time of at least about 30 minutes,
the wash solution comprises about 0.34 M sodium chloride, about 1 mM ethylmaleimide, about 25 mM ethylenediaminetetraacetic acid disodium salt, and combinations thereof, and
the filtering the depolymerized protein dispersion is done at a flow rate ranging from about 10 mL/min to about 70 mL/min and at a temperature ranging from about 0°C to about 8°C.

6. The method for producing the extracellular matrix hydrogel according to any one of claims 1 to 5,

wherein the decellularized tissue mesh comprises proteins having glycosylated protein tails, and
wherein the depolymerized protein dispersion comprises proteins having at least about 95 % of an amount of glycosylated protein tails in comparison to the decellularized tissue mesh, on a mass basis.

7. The method for producing the extracellular matrix hydrogel according to any one of claims 1 to 6, wherein the chaotropic agent comprises at least one of sodium dodecyl sulfate, guanidinium chloride, guanidinium thiocyanide, urea, guanidine, thiourea, salts of calcium, nitrates, dithiothreitol, mercaptoethanol, tributyl phosphine, sodium borohydride, lithium perchlorate, lithium acetate, magnesium chloride, and combinations thereof.

8. The method for producing the extracellular matrix hydrogel according to any one of claims 1 to 7, wherein separating the homogenized mixture into the waste precipitate and the supernatant comprises centrifugation, and/or wherein the filtering is performed at a temperature of about 4°C.

9. The method for producing the extracellular matrix hydrogel according to claim 2, wherein at least one of:

the halogenated solvent is chloroform;
the halogenated solvent is mixed with the sol at a concentration ranging from about 0.4 % v/v to about 3 % v/v, and
the halogenated solvent is mixed with the sol for a time period ranging from about 3 hours to about 12 hours.

10. The method for producing the extracellular matrix hydrogel according to claim 2, wherein at least one of:

the hygroscopic agent comprises a potassium citrate, and
the extracellular matrix hydrogel has a stiffness ranging from about 1 kPa to about 250 kPa.

11. The method for producing the extracellular matrix hydrogel according to any one of claims 1 to 10, wherein the extracellular matrix hydrogel comprises at least one of:

    a primary human hepatocytes adhesion value ranging from about 1 kPa to about 10 kPa;
    an FAK expression induction value ranging from about to 2 fold about 10 fold;
    the extracellular matrix hydrogel has a toxicity rate of less than about 1 % in mice; and
    an orthotropic tumor growth rate ranging from about 10 mm$^3$/day to about 200 mm$^3$/day.

12. The method for producing the extracellular matrix hydrogel according to any one of claims 1 to 11, further comprising:
    harvesting the tissue sample from the tumor; and/or
    mechanically homogenizing the depolymerizing mixture at a temperature of about 4 °C.

13. The method for producing the extracellular matrix hydrogel according to any one of claims 1 to 12, wherein at least one of:

    filtering comprises at least one of a double flow diafiltration, dialysis bag filtration, flow diafiltration, hollow fiber ultrafiltration, cartridge hollow fiber filtration, and cartridge tangential flow ultrafiltration;
    the yield of depolymerized protein dispersion is at least about 70 mg/mL (depending on the origin value can variate from 10 mg/mL for lipid-rich tissues to 110 mg/mL for heavily fibrotic tissue); and
    the chilled temperature ranges from about 0 °C to about 8°C.

14. The method for producing the extracellular matrix hydrogel according to any one of claims 1 to 13, wherein a filtration buffer used during the filtering comprises at least one of water, a sodium dodecyl sulfate at concentration ranging from about 0.5% by volume to about 2% by volume, and a sodium chloride at a concentration ranging from about 0.1 M to about 4 M.

15. The method for producing the extracellular matrix hydrogel according to any one of claims 1 to 14,

    wherein the filtering the depolymerized protein dispersion is done at a temperature ranging from about 0°C to about 8°C, and

wherein gelling further comprises raising a temperature of the sol from the temperature ranging from about 0 °C to about 8°C to a temperature ranging from about 26 °C to about 50°C for a time period of at least about 1 minute.

105

110

115

Mincing,
Filtering with
100 micron mash

Decellularization

120

Double-flow diafiltration,
CHCl3 sterilization at +4°C

Sterile, stable
ECM sol

125

Dispensing into vials,
Cryopreservation

130

Chaotrope-induced
swelling and micronization,
Centrifugation

Gelation on the surface

135

FIG. 1

FIG. 2

Figure of the decellularized tissue fragment with PI staining and positive control before the decellularization

Before decellularization

# FIG. 3A

After decellularization

# FIG. 3B

FIG. 4

EP 4 684 813 A1

The GuaHCl concentration from time curve

FIG. 5

EP 4 684 813 A1

FIG. 6

Representative sketch of the working model of diafiltration reactor, containing of the cooling jacked, the manifold for dialysis membranes, inlet and outlet permeate flow, inlet and outlet retentate flow and the magnetic stirrer

FIG. 7

EP 4 684 813 A1

FIG. 8

FIG. 9A

FIG. 9B

(0.004 µm, 4.267 µm) 24.91 = 2.401e-06 nm

FIG. 9C

(0.424 µm, 0.002 µm) 2.87 deg

D - period = 61,75 ± 1,375 nm

FIG. 9D

EP 4 684 813 A1

FIG. 10A

EP 4 684 813 A1

Size Distribution by Intensity

FIG. 10B

FIG. 11

4T1 orthotopic tumors progression in Balb/c mice

FIG. 12A

Balb/c mice body weigh bearing 4T1 subcutaneus tumors

FIG. 12B

MDA-MB-231 subcutaneus tumors
progression in SCID mice

FIG. 13A

Body weigh of SCID mice bearing
MDA-MB-231 orthotopic tumors

FIG. 13B

Europäisches
Patentamt

European
Patent Office

Office européen
des brevets

# EUROPEAN SEARCH REPORT

**Application Number**

EP 25 19 0194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 829 000 A (KLEINMAN HYNDA K [US] ET AL) 9 May 1989 (1989-05-09) * example 1 * * column 4, line 27 - line 29 * ----- | 1,2,4-9, 12-15 | INV. A61L27/36 A61L27/52 C12N5/00 |
| A | WO 2016/146893 A1 (UNIV OF OULU [FI]) 22 September 2016 (2016-09-22) * claims 7, 11 * ----- | 1-15 | |
| A | WANG XUJIE ET AL: "Strengthening gelatin hydrogels using the Hofmeister effect", SOFT MATTER, vol. 17, no. 6, 8 December 2020 (2020-12-08), pages 1558-1565, XP093338023, GB ISSN: 1744-683X, DOI: 10.1039/D0SM01923B * section 2.2; page 1, right-hand column; page 2, left-hand column * ----- | 1,3,10 | |

TECHNICAL FIELDS
SEARCHED (IPC)

A61L
C12N

The present search report has been drawn up for all claims

| Place of search | Date of completion of the search | Examiner |
|---|---|---|
| The Hague | 20 November 2025 | Lopez Serrano, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 0194

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2025

| Patent document cited in search report | | Publication date | Patent family member(s) | | Publication date |
|---|---|---|---|---|---|
| US 4829000 | A | 09-05-1989 | AT | E66247 T1 | 15-08-1991 |
| | | | CA | 1291432 C | 29-10-1991 |
| | | | EP | 0218065 A2 | 15-04-1987 |
| | | | JP | H0740932 B2 | 10-05-1995 |
| | | | JP | S62129222 A | 11-06-1987 |
| | | | US | 4829000 A | 09-05-1989 |
| WO 2016146893 | A1 | 22-09-2016 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- UA A202403745